# EUROPEAN PATENT APPLICATION

(11) **EP 3 461 487 A1**
(43) Date of publication of application: **03.04.2019**
(21) Application number: 17382649.6
(22) Date of filing: 29.09.2017
(51) Int. Cl.: A61K 31/7105, C07H 21/02, C12N 15/11, C12N 15/87

(54) **COMPOSITIONS AND METHODS FOR THE DELIVERY OF MRNA TO HEPATIC CELLS**

(71) Applicant: Nlife Therapeutics S.L., 18100 Armilla, Granada (ES)
(72) Inventor: MONTEFELTRO, Andrés Pablo, 18100 Armilla, Granada (ES); REVILLA SÁNCHEZ, Raquel, 18100 Armilla, Granada (ES); JIMÉNEZ SÁNCHEZ, Laura, 18100 Armilla, Granada (ES); PARIS, Clément, 18100 Armilla, Granada (ES); TURRADO GARCÍA, Carlos, 18100 Armilla, Granada (ES); ALVARADO URBINA, Gabriel, 18100 Armilla, Granada (ES)
(74) Representative: ABG Intellectual Property Law, S.L.

(57) **Abstract**

The invention provides a composition for selective delivery of an mRNA to a liver cell comprising (i) an mRNA of interest and (ii) one or more conjugates comprising a nucleic acid at least in part substantially complementary to a region of the mRNA, and at least one liver targeting moiety attached to the nucleic acid. The liver targeting moiety comprises at least one selective agent that binds specifically to one or more proteins on the surface of a liver cell. The compounds of the present invention are useful for the delivery of the mRNA of interest to a liver cell of interests where it can be translated into its encoded protein. Thus, the compound of the invention is useful for the treatment of diseases which require the expression of the protein encoded by the mRNA of interest in the target liver cell.

## Description

### FIELD OF THE INVENTION

The present invention relates to complexes comprising an mRNA of interest and a nucleic acid, the sequence of which binds to said mRNA of interest, and one or more targeting moieties attached to said nucleic acid. The targeting moieties have an affinity towards molecules on the surface of specific liver cells, thus allowing the delivery of the mRNA of interest to said specific liver cells.

### BACKGROUND ART

Gene therapy is a promising approach for treating diseases since it allows to effectively and sustainably expressing a therapeutic protein in a place of interest. Gene therapy can be achieved by the introduction of a DNA sequence of interest or of an mRNA of interest. Several techniques have been disclosed regarding DNA based gene therapy, such as virus-mediated delivery of DNA sequences. However, DNA delivery leads to the risk of random genomic integration or to the overexpression of the delivered gene.

Gene therapy through mRNA delivery involves no risk of random genome integration, and does not need nuclear entry for protein expression, which allows the synthesis of the protein in any cell and immediately after being introduced into the cell.

The liver is a vital organ that plays an important role in the detoxification of different metabolites, in protein synthesis, and in the production of biochemical compounds required for digestion. It also participates in the control of the metabolism, in the regulation of glycogen storage, in the decomposition of red blood cells and in hormone production. Thus, a proper and finely controlled function of the liver may contribute to the treatments of several diseases directly affecting the liver or any other organ or function of the body.

Karikó K. et al. (Journal of Neuroscience Methods 2001, 105: 77-86) have reported a method of mRNA delivery into the CNS allowing the expression of the mRNA encoded protein. Said method consisted on the intracerebral injection of lipofectamin-complexed mRNAs or lipid-complexed mRNAs. The authors showed that the encapsulated mRNA led to a higher expression of the encoded protein than naked mRNAs. However, the stability of the transfected mRNAs was almost null at 24 h post-transfection leading to a very punctual expression of the encoded protein.

Akitsugu M. et al. (Sci Rep. 2015, 5:15810) described a method for introducing mRNA into the liver. Said method consists on hydrodynamic intravenous injection of a synthesized polymer-based carrier of polyplex nanomicelles. The authors indicate that mRNA delivery induced efficient protein expression in almost 100% of liver cells. Thus, this method does not allow targeting specific hepatic cells, and additionally, it also requires the use invasive techniques, such hydrodynamic intravenous injections in mouse under anesthesia.

Therefore, there is a need in the art for compositions which allow the delivery of mRNAs of interest specifically into specific hepatic cells that overcome the defects of the methods known in the art.

### SUMMARY OF THE INVENTION

The inventors have developed compositions that comprise a nucleic acid that hybridizes with a region of an mRNA of interest, wherein the nucleic acid is linked to one or more selective ligands for one or more receptors expressed in target hepatic cells. Said receptor/s can be endocytosed in response to the binding of the selective ligand. These compositions are shown to be particularly useful for the delivery of the mRNA of interest to the interior of the hepatic cell expressing the receptor/s. Without wishing to be bound by any theory, it is believed that the ligand will bind to the corresponding receptor/s in the surface of the liver cell wherein the receptor/s is/are expressed. This will in turn result in the translocation of the complex consisting of the nucleic acid-mRNA to the interior of the liver cell by means of receptor-mediated endocytosis.

The results provided in the present invention illustrate that the mechanism used by the cells to signal via surface receptor are adequate means for promoting delivery to cells of small molecules attached to molecules showing affinity for said receptors.

The mRNA delivery system according to the invention has some advantages. Firstly, it is specifically targeted to cells wherein the protein receptor is expressed, avoiding side effects due to the expression of the protein in undesired locations. Secondly, the expression of the mRNA can be further regulated once introduced in the target cell by introducing regulatory sequences in the mRNA of interest.

Accordingly, in a first aspect, the invention relates to a composition for selective delivery of an mRNA to a liver cell comprising:
a) an mRNA and
b) one or more conjugates having the general formula:

   N-Tx
wherein N is a nucleic acid, the sequence of which is at least in part substantially complementary to a region of the mRNA, T is a liver targeting moiety comprising at least one selectivity agent S which binds specifically to one or more proteins on the surface of a liver cell and x is an integer which is equal to or higher than 1, wherein a complex is formed between the mRNA and the nucleic acid N by base pairing between the region within the mRNA which is complementary to the nucleic acid and the nucleic acid forming part of the conjugate.

In a further aspect, the invention relates to a method for preparing a complex according to the invention comprising contacting the mRNA and the conjugate or conjugates under conditions adequate for hybridization between the mRNA and the nucleic acid or nucleic acids within the conjugate or conjugates.

In additional aspects, the invention relates to a complex according to the invention for use in the treatment of a disease which requires the expression in the liver of the protein encoded by the mRNA forming part of the complex.

These and other objects of the present invention will be further described in the detailed description section that follows, and they are not intended to be limiting of the present invention. Unless otherwise defined, all technical and scientific terms used herein have the same meaning as commonly understood by one ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps.

### BRIEF DESCRIPTION OF THE DRAWINGS

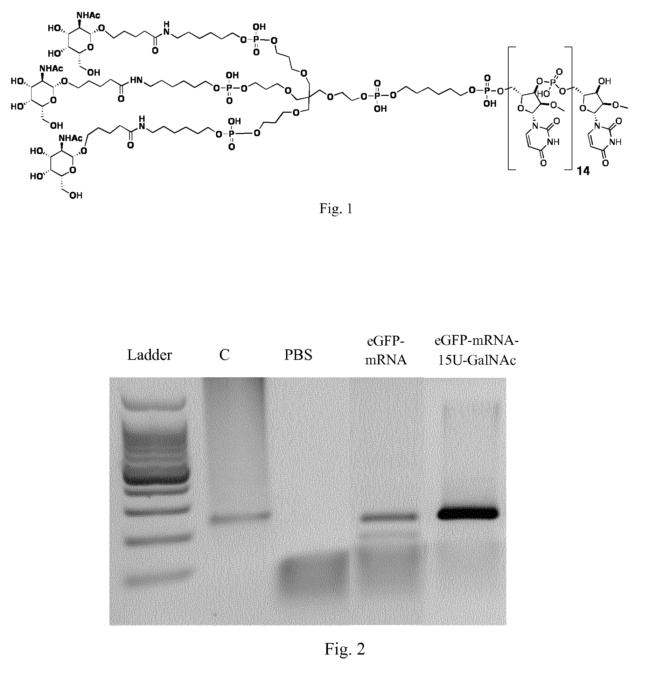

**Figure 1** shows the structure of the PolyU GalNAc compound.
**Figure 2** shows an agarose gel electrophoresis showing the results of the RT-PCR obtained from the RNA extracted from fresh liver of treated mice with PBS (buffer control), eGFP-mRNA and eGFP-mRNA-15U-GalNAc complex at a concentration of 100 ng in single dose and sacrificed 72 hrs later. Animals treated with eGFP-mRNA and eGFP-mRNA-15U-GalNAc complex showed a band corresponding to the specific RT-PCR fragment (250 pb) with a higher accumulation in liver tissue when the mRNA was modified with 15U-GalNAc. Line 1: size ladder, line 2: C, RT-PCR of eGFP-mRNA as control of the reaction.

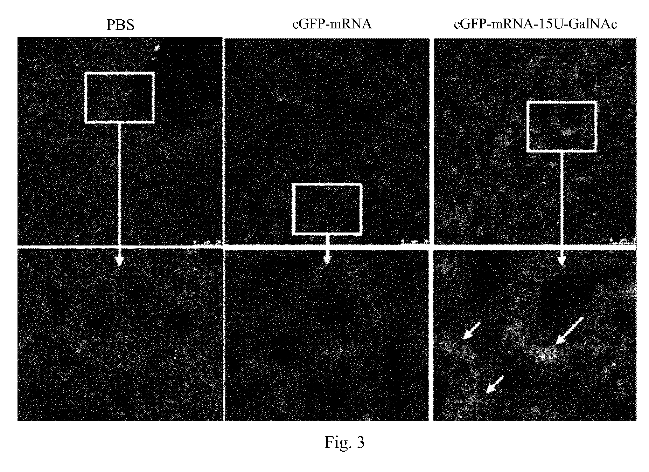

**Figure 3** shows confocal microscope images of liver sections of animals treated with PBS, eGFP-mRNA or eGFP-mRNA-15U-GalNAc complex at a concentration of 100 ng in single dose and sacrificed 72 hrs later. Mice treated with targeted eGFP-mRNA showed protein expression of eGFP (arrows) in liver cells (hepatocytes). Lower panels show magnified areas shown as squares in upper panel.

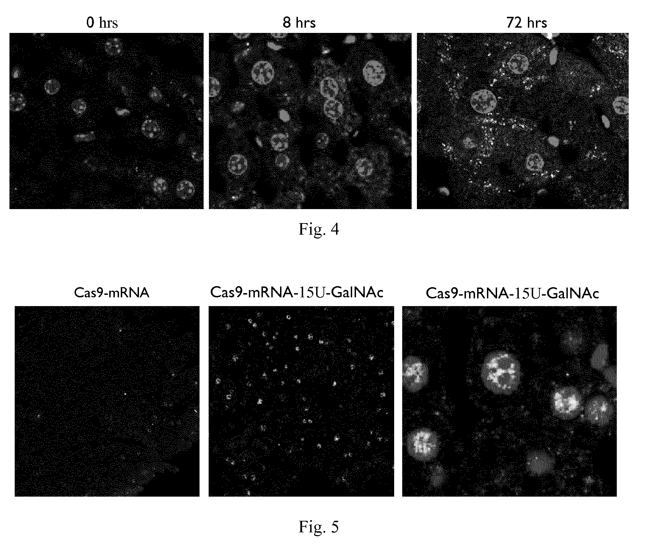

**Figure 4** shows confocal microscope images of liver sections of animals treated with PBS (0hrs) or eGFP-mRNA-15U-GalNAc complex at a concentration of 100 ng in single dose and sacrificed 8 or 72 hrs after administration. Sections were counterstained with DAPI to visualize the nuclei. Protein expression of eGFP was observed at 8 hrs with a strong cytoplasmic accumulation at 72 hrs (arrows).
**Figure 5** shows confocal microscope images of liver sections of mice treated with Cas9-mRNA (left panel) or Cas9-mRNA-15U-GalNAc complex (middle and right panel) at a concentration of 900 ng (2 doses of 450 ng each in consecutive days) intravenously and sacrified 72 hrs later. Right panel shows a higher magnification image co-stained with the nuclear marker DAPI showing that Cas9 is mainly localized in the cellular nucleus.

### DETAILED DESCRIPTION OF THE INVENTION

The authors of the present invention have observed that it is possible to specifically deliver an mRNA of interest to a liver cell by coupling the mRNA to a nucleic acid which is capable of at least partially hybridizing to a region of said mRNA, wherein said nucleic acid is conjugated to a ligand that binds specifically to one or more proteins on the surface of a liver cell. Additionally, the authors of the present invention have observed that said mRNA can be translated in said liver cell, thereby leading to the expression of the said protein encoded mRNA in the target liver cell.

### A. Complex of the invention

In a first aspect, the invention relates to a complex for selective delivery of an mRNA to a liver cell comprising:
a) an mRNA and
b) one or more conjugates having the general formula:

   N-Tx
wherein N is a nucleic acid, the sequence of which is at least in part substantially complementary to a region of the mRNA, T is a liver targeting moiety comprising at least one selectivity agent S which binds specifically to one or more proteins on the surface of a liver cell and x is an integer which is equal to or higher than 1, wherein a complex is formed between the mRNA and the nucleic acid N by base pairing between the region within the mRNA which is complementary to the nucleic acid and the nucleic acid forming part of the conjugate.

### A. 1. mRNA of the complex of the invention

The term "mRNA", as used herein, refers to a messenger RNA as understood by a skilled person in the art, which can be translated into its encoded protein. The mRNA may contain 5' and 3' structures that stabilize the mRNA and/or enhance its translation.

A typical, but not limiting structure in the 5' end of the mRNA, is the anti-reverse cap analog (ARCA) 3'-O-Me-m7G(5')ppp(5')G. Additional non-limiting examples of 5' cap analogs include: standard cap analog (m7G(5')ppp(5')G); unmethylated cap analog (such as G (5')ppp(5')G); methylated cap analog for A+1 sites (m7G(5')ppp(5')A); unmethylated cap analog for A+1 sites (G(5')ppp(5')A); inverted abasic residue (moiety), 4',5'-methylene nucleotide; 1 -(beta-D-erythrofuranosyl) nucleotide, 4'-thio nucleotide, carbocyclic nucleotide; 1,5-anhydrohexitol nucleotide; L-nucleotides; alpha- nucleotides; modified base nucleotide; phosphorodithioate linkage; threo-pentofuranosyl nucleotide; acyclic 3',4'-seco nucleotide; acyclic 3,4-dihydroxybutyl nucleotide; acyclic 3,5-dihydroxypentyl nucleotide, 3 '-3 '-inverted nucleotide moiety; 3'-3'-inverted abasic moiety; 3'-2'-inverted nucleotide moiety; 3'-2'inverted abasic moiety; 1,4-butanediol phosphate; 3'-phosphoramidate; hexylphosphate; aminohexyl phosphate; 3 '-phosphate; 3 '-phosphorothioate; phosphorodithioate; or bridging or non-bridging methylphosphonate moiety. Details are described in WO97/26270, incorporated by reference herein.

A typical, but non-limiting structure located on the 3' end of the mRNA is a poly(A) tail. The poly(A) tail consists of multiple adenosine monophosphates; in other words, it is a stretch of RNA composed by adenine bases. The poly(A) tail is important for the nuclear export, translation, and stability of the mRNA. The length of the poly(A) tail of mRNAs can vary broadly, from at least 10 nt, at least 12 nt, at least 15 nt, at least 20 nt to less than 100 nt, less than 150 nt, less than 200 nt, less than 250 nt in length (Kojima S. et al., Genes and Dev. 2012; 26(24): 2724-2736).

The mRNA of the complex of the invention may contain one or more modifications in the nucleobases, in the sugars and/or in the internucleotide linkages. Modifications to one or more backbone residues of the nucleic acids may comprise one or more of the following: 2' sugar modifications such as 2'-O-methyl (2'-OMe), 2'-O-methoxyethyl (2'-MOE), 2'-O-methoxyethoxy, 2'- Fluoro (2'-F), 2'-AIIyI, 2'-O-[2-(methylamino)-2-oxoethyl], 2'-O-(N-methylcarbamate); 4' sugar modifications including 4'-thio, 4'-CH₂-O-2'-bridge, 4-(CH₂)₂-O-2'-bridge; Locked Nucleic Acid (LNA); Peptide Nucleic Acid (PNA); Intercalating nucleic acid (INA); Twisted intercalating nucleic acid (TINA); Unlocked nucleic acid (UNA); Hexitol nucleic acids (HNA); arabinonucleic acid (ANA); cyclohexane nucleic acids (CNA); cyclohexenylnucleic acid (CeNA); threosyl nucleic acid (TNA); Morpholino oligonucleotides; Gap-mers; Mix-mers; Incorporation Arginine-rich peptides; addition of 5'-phosphate to synthetic RNAs; RNA Aptamers (Que-Gewirth NS, Gene Ther. 2007 Feb;14(4):283-91.); RNA Aptamers regulated with antidotes on the subject of the specific RNA aptamer (Oney S. et al., Oligonucleotides. 2007 Fall;17(3):265-74) or any combinations thereof.

In a preferred embodiment of the invention, the mRNA of the complex of the invention contains:
a) an anti-reverse cap analog (ARCA),
b) one or more nucleosides selected from the group consisting of 2'-0-methyl nucleoside, 2'-fluoronucleoside, pseudouridine, N-methyl pseudouridine, 2-thiouridine, 5-methylcytidine, 6-methyl adenosine and inosine, or
c) a combination of a) and b).

Preferably, the mRNA of the complex of the invention encodes a protein that treats, or contributes to the treatment, of a disease. In a more preferred embodiment, the treatment of the disease can be mediated by the expression in the liver of the protein encoded by the mRNA forming part of the complex. In another embodiment, the mRNA of the invention encodes a protein which is naturally expressed in a liver cell. Non-limiting examples of mRNAs include mRNAs encoding the following proteins: erythropoietin, alpha-1 antitrypsin, alpha-L-iduronidase, iduronate-2-sulfatase, galactosamine-6 sulfatase, beta-galactosidase, N-acetylgalactosamine-4-sulfatase, chondroitinsulfatase, chondroitinase, acetylgalactosamine 4-sulfatase, N-acetylgalactosamine 4-sulfate sulfohydrolase, EC 3.1.6.12, beta-glucuronidase, acid alpha-glucosidase, glucocerebrosidase, adenosine deaminase, Ornithine transcarbamylase, N-acetylglutamate synthase, Carbamoyl phosphate synthetase, Argininosuccinate synthase, Argininosuccinate lyase, Arginase, lipase A, lysosomal acid, cholesterol esterase, ceramidase, alpha galactosidase A, alpha-N-acetylgalactosaminidase, acid alpha-glucosidase or Factor I, II, V, VII, VII, IX, X, XI, XII and XIII, monoclonal therapeutic Antibodies.

The mRNA of the complex of the invention may contain 5' and a 3' untranslated regions (5'- and 3'UTR).

In a further embodiment, a nucleotide sequence is introduced in the open reading frame of the mRNA of the complex of the invention so that it is translated together with the mRNA of the complex of the invention. As a result, the peptide encoded by the mRNA of the complex of the invention and that encoded by the introduced sequence form a unique polypeptide. Once the mRNA of the complex is translated, the peptide encoded by the introduced sequence can regulate the localization, stability, transport or post-translational modifications, or a combination thereof, of the protein encoded by the mRNA of the complex of the invention. As non-limiting examples, said introduced sequence comprises a sequence encoding a nuclear localization signal (NLS), or a cell penetrating peptide, or a combination thereof. In a preferred embodiment, the amino acid sequence of the NLS selected from the list consisting in SEQ ID no:3-20, or a combination thereof. In another preferred embodiment, the amino acid sequence of the CPP is selected from the ist consisting on SEQ ID no.: 21-136, or a combination thereof.

### A.2. Conjugate of the invention

The term "conjugate", as used herein, refers to any compound resulting from the covalent attachment of two or more individual compounds. In the present invention, conjugate refers to a molecule comprising a nucleic acid N and at least one or more targeting moieties T, which themselves comprise one or more selectivity agents S. The one or more targeting moieties T and the nucleic acid N are covalently coupled, being said coupling direct or via a linking compound. Additionally, the selectivity agent/s S of the conjugate of the invention can bind specifically to one or more proteins on the surface of a liver cell, and the nucleic acid N of the conjugate of the invention is at least in part substantially complementary to the mRNA of the invention.

The terms "covalent coupling" or "covalent attachment" mean that the nucleic acid N and the one or more targeting moieties T are directly covalently joined between each other, or indirectly covalently joined between each other through an intervening moiety or moieties, such as a linker, or a bridge, or a spacer, moiety or moieties.

### A2.1 Nucleic acid of the conjugate of the invention (N)

The nucleic acid N consists on a nucleic acid polymer that comprises a single stranded sequence that is substantially complementary to a sequence of the mRNA of the invention.

Thus, the nucleic acid of the conjugate of the invention and the mRNA of the complex of the invention are at least in part substantially complementary.

The term "nucleic acid", as used herein, refers to a polymer having two or more deoxyribonucleotide, ribonucleotide or nucleotide analog molecules as well as molecules that are structurally similar to a native nucleic acid, but differ from the native nucleic acid (e.g., through chemical modification) at one or more of the nucleic acid backbone (e.g., phosphate in native nucleic acids), nucleic acid sugar (e.g., deoxyribose for native DNA and ribose in native RNA), and nucleic acid base (e.g., adenosine, cytosine, guanine, thymidine, or purine in native nucleic acids).

The term "substantially complementary", as used herein, refers to base pair complementarity between a certain number of the nucleotides of the sequence from the nucleic acid N involved in the base pairing and a target sequence of the mRNA of interest. In this sense, a sequence is substantially complementary to the mRNA of the complex of the invention when at least 50%, at least 60%, at least 70%, at least 80%, at least 90% or 100% of the nucleotides of the sequence do base pair with nucleotides comprised in the target sequence of the mRNA of the complex of the invention. In an additional embodiment, at least 50%, at least 60%, at least 70%, at least 80%, at least 90% or 100% of the nucleotides of the target sequence of the mRNA of the complex of the invention do base pair with nucleotides comprised in the nucleic acid N of the conjugate of the invention. The length of the sequence of the nucleic acid N involved in the base pairing with the mRNA of interest can vary as long as the resulting complex is stable enough. In preferred embodiments the length of the sequence of the nucleic acid N of the conjugate that shows complementarity with the mRNA is of at least 5 nt, at least 10 nt, at least 15 nt, at least 20 nt, at least 25 nt, at least 30 nt, at least 35 nt, to less than 100 nt, less than 150 nt, less than 200 nt, less than 250 nt. A preferred embodiment of the invention refers to the composition of the invention, wherein the the sequence of the nucleic acid N of the conjugate that shows complementarity with the within one or more of the conjugates comprises at least 15 nucleotides. Similarly, the length of the sequence from the mRNA region involved in the base pairing with the nucleic acid of the conjugate of the invention can vary from at least 5 nt, at least 10 nt, at least 15 nt, at least 20 nt, at least 25 nt, at least 30 nt, at least 35 nt, to less than 100 nt, less than 150 nt, less than 200 nt, less than 250 nt. Another preferred embodiment of the invention refers to the composition of the invention, wherein the mRNA region involved in the base pairing with the nucleic acid of the conjugate of the invention comprises at least 15 nucleotides.

In a preferred embodiment, more than one nucleic acid N hybridizes with the mRNA of the invention. In a further preferred embodiment, the number of nucleic acids N that hybridize with the mRNA of the complex of the invention are at least 2, at least 3, at least 4, at least 5, at least 6, at least 7, at least 8, at least 9, at least 10, at least 15, at least 20, at least 25, at least 30.

If the nucleic acid N of the conjugate of the invention hybridises with the mRNA only through part of its sequence, then after base-pairing with the mRNA, the nucleic acid may show non-complementary overhanging ends, located at the 5' end, at the 3' end or at both ends of the nucleic acid. Alternatively, the nucleic acid N of the conjugate of the invention may show anon-complementary region not located at the 5' or 3' ends. In another alternative, the nucleic acid N may show more than one non-complementary regions located at any of these locations or at a combination thereof.

The length of the non-complementary region may be of at least 1, at least 2, at least 3, at least 4, at least 5, at least 6, at least 7, at least, 8 at least 9, at least 10 at least 12, at least 15, at least 18 at least 20, at least 22, at least 25 nucleotides. In a preferred embodiment, the length of the non-complementary region is of at least 1 nucleotide. In a further preferred embodiment, the length of the non-complementary region is of at least 5 nucleotides long. In yet a further preferred embodiment, the length of the non-complementary region is of at least 10 nucleotides.

The nucleic acid polymer of the invention can consist on an RNA oligonucleotide or a DNA oligonucleotide.

The nucleic acid of the invention may contain one or more modifications in the nucleobases, in the sugars and/or in the internucleotide linkages. Modifications to one or more backbone residues of the nucleic acids may comprise one or more of the following: 2' sugar modifications such as 2'-O-methyl (2'-OMe), 2'-O-methoxyethyl (2'-MOE), 2'-O-methoxyethoxy, 2'- Fluoro (2'-F), 2'-AIIyI, 2'-O-[2-(methylamino)-2-oxoethyl], 2'-O-(N-methylcarbamate); 4' sugar modifications including 4'-thio, 4'-CH₂-O-2'-bridge, 4-(CH₂)₂-O-2'-bridge; Locked Nucleic Acid (LNA); Peptide Nucleic Acid (PNA); Intercalating nucleic acid (INA); Twisted intercalating nucleic acid (TINA); Unlocked nucleic acid (UNA); Hexitol nucleic acids (HNA); arabinonucleic acid (ANA); cyclohexane nucleic acids (CNA); cyclohexenylnucleic acid (CeNA); threosyl nucleic acid (TNA); Morpholino oligonucleotides; Gap-mers; Mix-mers; Incorporation Arginine-rich peptides; addition of 5'-phosphate to synthetic RNAs; RNA Aptamers (Que-Gewirth NS, Gene Ther. 2007 Feb;14(4):283-91.); RNA Aptamers regulated with antidotes on the subject of the specific RNA aptamer (ref. Oney S, Oligonucleotides. 2007 Fall;17(3):265-74.) or any combinations thereof.

Modifications to one or more internucleotide linkages of the nucleic acids may comprise one or more of the following: Phosphorothioate, phosphoramidate, phosphorodiamidate, phosphorodithioate, phosphoroselenoate, phosphorodiselenoate, phosphoroanilothioate and phosphoranilidate, or any combinations thereof.

In one embodiment of the invention, the nucleic acid polymer comprises Locked Nucleic Acid (LNA) sequences, often referred to as inaccessible RNA, which are modified RNA nucleotides. The ribose moiety of an LNA nucleotide is modified with an extra bridge connecting the 2' and 4' carbons (02',C4'-methylene bridge). The bridge "locks" the ribose in the 3'-endo structural conformation, which is often found in the A-form of DNA or RNA. LNA nucleotides can be mixed with DNA or RNA bases in the nucleic acid whenever desired. Such oligomers are commercially available. The locked ribose conformation enhances base stacking and backbone pre-organization. This significantly increases the thermal stability (melting temperature) and hybridization affinity of LNA-modified nucleic acids, besides having improved mismatch discrimination abilities. These properties make them very useful for antisense-based techniques.

In a preferred embodiment, the nucleic acid N comprises LNA nucleotides. In a more preferred embodiment, the sequence of the nucleic acid N that hybridizes with the mRNA of the complex of the invention comprises LNA nucleotides. In a more preferred embodiment, the nucleic acid N hybridizes with a region of the poly(A) tail of the mRNA of the complex of the invention and the sequence hybridizing with the poly(A) tail comprises LNA nucleotides. Preferably, the nucleic acid N hybridizes with a region of the poly(A) tail of the mRNA of the complex of the invention and the sequence hybridizing with the poly(A) tail comprises LNA nucleotides combined with uridines and/or thymidines nucleotides. In a further preferred embodiment, the nucleic acid N hybridizes with a region of the poly(A) tail of the mRNA of the complex of the invention and the sequence hybridizing with the poly(A) tail comprises LNA nucleotides that alternate with uridines and/or thymidine nucleotides.

Since the thermal stability, the hybridization affinity and the mismatch discrimination of LNA-comprising nucleic acids is higher than that of nucleic acids with no modified nucleotides, a base pairing sequence in the nucleic acid N comprising LNA modified nucleotides can comprise less complementary nucleotides but be equally efficient than a base pairing sequence with no modified nucleotides. Thus, the length of the sequence of the nucleic acid N of the conjugate of the invention comprising LNA modified nucleotides and base paring with a region of the mRNA of the complex of the invention can be shorter than an equivalent sequence with no modified nucleotides. The length of said sequences of the nucleic acid N can be of at least 5 ntd, at least 10 ntd, at least 12 ntd, at least 15 ntd, at least 20 ntd, at least 25 ntd, at least 30 ntd, at least 35 ntd. More preferably, the length of the sequence of the nucleic acid N of the conjugate of the invention comprising LNA modified nucleotides and base paring with a region of the mRNA of the complex of the invention is of at least 15 ntd length. Even more preferably, said sequence comprises uridine or thymidine nucleotides that alternate with LNA modified nucleotides.

In another embodiment, the nucleic acid polymer comprises Peptide Nucleic Acid (PNA) sequences, which are artificially synthesized polymers similar to DNA or RNA and are used in biological research and medical treatments. PNA is not known to occur naturally. DNA and RNA have a deoxyribose and ribose sugar backbone, respectively, whereas PNA's backbone is composed of repeating N-(2-aminoethyl)-glycine units linked by peptide bonds. The various purine and pyrimidine bases are linked to the backbone by methylene carbonyl bonds. PNAs are depicted like peptides, with the N- terminus at the first (left) position and the C-terminus at the right. Since the backbone of PNA contains no charged phosphate groups, the binding between PNA/DNA strands is stronger than between DNA/DNA strands due to the lack of electrostatic repulsion. Mixed base PNA molecules are true mimics of DNA molecules in terms of base-pair recognition. PNA/PNA binding is stronger than PNA/DNA binding.

In a preferred embodiment, the nucleic acid N comprises a PNA backbone. In a more preferred embodiment, the sequence of the nucleic acid N that hybridizes with the mRNA of the complex of the invention comprises a PNA backbone. In a yet more preferred embodiment, the nucleic acid N hybridizes with a region of the poly(A) tail of the mRNA of the complex of the invention and the sequence hybridizing with the poly(A) tail comprises a PNA backbone. Preferably, the nucleic acid N hybridizes with a region of the poly(A) tail of the mRNA of the complex of the invention and the sequence hybridizing with the poly(A) tail comprises uridines and/or thymidines nucleotides linked by a PNA backbone. In a further preferred embodiment, the nucleic acid N hybridizes with a region of the poly(A) tail of the mRNA of the complex of the invention and the sequence hybridizing with the poly(A) tail comprises thymidines linked by a PNA backbone.

Due to the higher binding strength of the nucleic acid polymers comprising PNA sequences, it is not necessary to design long PNA oligomers for use in base pair recognition processes. Thus, in a preferred embodiment, the sequence of the nucleic acid N that hybridizes with the mRNA of the complex of the invention comprising a PNA backbone can comprise less complementary nucleotides but be equally efficient than a base pairing sequence with no modified nucleotides nor a deoxyribose or ribose sugar backbone. Thus, the length of the sequence of the nucleic acid N of the conjugate of the invention comprising a PNA backbone and base paring with a region of the mRNA of the complex of the invention can be shorter than an equivalent sequence with no modified nucleotides nor a deoxyribose or ribose sugar backbone. The length of said sequences of the nucleic acid N can be of at least 5 ntd, at least 10 ntd, at least 12 ntd, at least 15 ntd, at least 20 ntd, at least 25 ntd, at least 30 ntd, at least 35 ntd. More preferably, the length of the sequence of the nucleic acid N of the conjugate of the invention comprising a PNA backbone and base paring with a region of the mRNA of the complex of the invention is of at least 10 ntd length. Even more preferably, said sequence comprises uridine or thymidine nucleotides linked by a PNA backbone.

Alternatively, the nucleic acid polymer of the invention comprises morpholino sequences, which are synthetic molecules resulting from a redesign of the natural nucleic acid structure. Structurally, the difference between morpholinos and DNA or RNA is that while morpholinos have standard nucleobases, those bases are bound to 6-membered morpholine rings instead of deoxyribose/ribose rings and non-ionic phosphorodiamidate intersubunit linkages replace anionic phosphodiester linkages. Morpholinos are sometimes referred to as PMO (phosphorodiamidate morpholino oligonucleotide). The 6-membered morpholine ring has the chemical formula O-(CH₂-CH₂)₂-NH.

In another embodiment, the nucleic acid N of the conjugate of the invention comprises double stranded regions resulting from the interaction between different sequences of the same nucleic acid polymer.

In a particular embodiment, the nucleic acid of the conjugate of the invention is an RNA polymer comprising double stranded regions resulting from the interaction between different sequences of the same RNA polymer. In a further embodiment, the nucleic acid invention is a DNA polymer comprising double stranded regions resulting from the interaction between different sequences of the same DNA polymer.

The nucleic acid of the invention may comprise one or more molecules attached to any of its nucleotides, preferably at the 3' end of the nucleic acid polymer, allowing detecting the nucleic acid within the target cell, organ or organism. Said detectable molecule is preferably a detectable protein or fragment thereof. In this sense, a particular embodiment refers to the nucleic acid N of the conjugate of the invention to which a detectable moiety is attached. Said detectable moiety allows to detect the whole composition of the invention in the target cell, organ or organism. By inference, said detectable molecule can be attached to any of the molecules of the composition of the invention, permitting the detection of the molecule of the conjugate to which it is attached with similar techniques. Thus, the detectable molecule attached to any of the molecules of the complex of the invention permits to detect the whole complex of the invention in the target cell, organ or organism.

The techniques used to visualize the detectable molecule are known in the art, such as electromicroscopy, confocal microscopy, immunohistochemistry, Enzyme-Linked ImmunoSorbent Assay (ELISA), Western blot or Flow-cytometry. By inference, the detectable molecule attached to the nucleic acid of the invention or to any other molecule of the composition of the invention permits to detect the whole composition of the invention in the target cell, organ or organism.

In a preferred embodiment, the detectable molecule referred herein is selected from the list consisting on fluorochromes, enzymes, epitopes for binding a second binding reagent, for example an antigen or a member of a binding pair, such as biotin. In a further preferred embodiment the detectable molecule is fluorescein amidite (FAM).

In a further preferred embodiment, the nucleic acid N of the conjugate of the invention comprises a thymidine nucleotide or a polythymidine sequence in the 5'-and/or in the 3'-end and/or in an internal region. The number of thymidines forming the polythymidine sequence can be of at least 2, at least 3, at least 5, at least 6, at least 7, at least 10, at least 15, at least 20. In another embodiment, the nucleic acid N of the conjugate of the invention comprises several thymidine nucleotides or several polythymidine nucleotide sequences at different positions. In preferred embodiments, the nucleic acid N contains a thymidine at the 5' end and a thymidine at the 3' end, the nucleic acid N contains a polythymidine at the 5' end and a thymidine at the 3' end, the nucleic acid N contains a thymidine at the 5' end and a polythymidine at the 3' end, the nucleic acid N contains a polythymidine at the 5' end and a polythymidine at the 3' end.

Said thymidine or polythymidine nucleotide sequences can be used to attach at least one moiety of interest of the conjugate of the invention. A non-limiting example of chemical attachment between a thymidine and a moiety of interest is an attachment of a fluorescein moiety to the carbon 5 of the thymidine ring by a 6-carbon spacer arm. Said moiety/ies can be targeting moiety/ies, cell penetrating peptide/s, nuclear localization signal/s, and/or detectable molecule referred herein, or a combination thereof.

### A.2.2 Targeting moiety T of the conjugate of the invention

The term "targeting moiety", as used herein, refers to any substance which comprises at least one selectivity agent that binds to one or more specific proteins expressed in a liver cell. This binding specificity allows the delivery of the molecule attached to said targeting moiety (comprising the selectivity agent/s) to the liver cell, tissue or organ containing said specific protein to which the selectivity agent/s bind/s. In this way, a conjugate carrying said targeting moiety will be directed specifically to said liver cells when administered to an animal or contacted *in vitro* with a population of cells of different types.

Since the selective agents of the targeting moieties bind to proteins present on the surface of a liver cell, they are liver cell selective agents. Thus, the targeting moieties of the conjugate of the invention, which comprise at least one of such liver cell selective agent S, are also liver cell targeting moieties. Thus, in a preferred embodiment, the conjugate of the invention comprises at least one liver cell targeting moiety T comprising at least one selectivity agent S that binds specifically to one or more proteins on the surface of a liver cell.

As used herein, specific binding of a first molecule to a second molecule refers to the ability of the first molecule to bind said second molecule in a way that is measurably different from a non-specific interaction. A selectivity agent according to the present invention may show a Kd for the target (the specific protein on the target cell) of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M or greater.

The specific protein expressed in the target liver cell is preferably a protein expressed in the surface of the liver cell, and that preferably undergoes endocytosis or alternative mechanisms allowing the integration of the complex that comprises the selectivity conjugate into the target cell. Non-limiting examples of such protein are: hepatic Asialoglycoprotein receptor (ASGPR), folate receptor, biotin receptor, retinoic acid receptor, glucose transporter isoform 1, surface antigens, integrins, growth factor receptors, transferrin receptor, glycyrrhetinic acid receptor, low-density lipoprotein receptor, scavenger receptor type B-1, serotonin receptor.

The conjugates of the invention comprise at least one targeting moiety T. Preferably, the conjugate of the invention comprises at least 2, at least 3, at least 4, at least 5, at least 6, at least 7, at least 8, at least 9, at least 10 targeting moieties. The targeting moiety or moieties are connected to the nucleic acid N of the conjugate of the invention via a terminal nucleotide position and/or via an internal nucleotide position. A "terminal nucleotide" as used herein refers to the first 5'-end nucleotide or to the last 3'-end nucleotide of a nucleic acid. An "internal nucleotide" as used herein refers to any nucleotide of a nucleic acid different from the terminal nucleotides just defined.

The targeting moiety may be connected with the nucleic acid N by a direct link between the targeting moiety and a nucleotide within the nucleic acid N. Alternatively, the targeting moiety may be connected with the nucleic acid N by a spacer molecule. A "spacer molecule" as used herein refers to any flexible part of a molecule providing a connection between two other parts of a molecule. Thus, in a preferred embodiment, the conjugate of the invention comprises at least one liver cell targeting moiety T comprising at least one selectivity agent S that binds specifically to one or more proteins on the surface of a liver cell wherein the targeting moiety T is connected to the nucleic acid N via a single spacer molecule to a terminal nucleotide and/or an internal nucleotide.

If the conjugate comprises a single targeting moiety T, the targeting moiety may be connected to the nucleic acid N via a spacer molecule to a terminal nucleotide and/or to an internal nucleotide. If the conjugate comprises more than one targeting moiety, at least one targeting moiety may be attached to a terminal nucleotide of the nucleic acid N (in the 5'- or in the 3'-end of the nucleic acid N). More specifically, in one embodiment one targeting moiety is attached to the 5'-end of the nucleic acid N and another targeting moiety is attached to the 3'-end of the nucleic acid N; in another embodiment, at least one targeting moiety is attached to a terminal nucleotide of the nucleic acid N (one targeting moiety attached to one of the terminal nucleotides, or one targeting moiety attached to each terminal nucleotide) and at least one additional targeting moiety is attached to an internal nucleotide. Alternatively, all the targeting moieties of the conjugate are attached to internal nucleotides.

In a preferred embodiment, the conjugate of the invention comprises one targeting moiety with a single selectivity agent. However, the targeting moiety T of the invention can comprise more than one selectivity agent S which can be the same or different. Thus, a more preferred embodiment of the invention refers to the complex of the invention wherein each targeting moiety comprises n selectivity agents S1 to Sn, wherein the selectivity agents S1 to Sn are the same or different. The letter n used herein is an integer which is equal to or higher than 1, preferably n is at least 2, at least 3, at least 4, at least 5, at least 6, at least 7, at least 8, at least 9, at least 10, being the selective agents within the same targeting moiety the same or different. A further preferred embodiment refers to the complex of the invention wherein n is equal to or higher than 2 and wherein the selectivity agents S1 to Sn are connected to the nucleic acid via a branched spacer molecule containing the selectivity agents S1 to Sn connected to different branches of the spacer molecule. More specifically, the selectivity agents S1 to Sn in the targeting moiety T can be connected in a serial manner, i.e. connected to each other forming a chain of selectivity agents or in parallel, i.e. connected to different positions within a spacer molecule.

In a further embodiment, the conjugate of the invention comprises more than one targeting moiety wherein the selective agents are the same within each targeting moiety. In yet another embodiment, the conjugate of the invention comprises more than one targeting moiety wherein the selective agents are different within each targeting moiety. In a further embodiment, the conjugate of the invention comprises more than one targeting moiety wherein the selective agents are the same within each targeting moiety and between targeting moieties. In another preferred embodiment, the conjugate of the invention comprises more than one targeting moiety wherein the selective agents are same within each targeting moiety but different between targeting moieties. In yet another embodiment, the conjugate of the invention comprises more than one targeting moiety wherein at least one targeting moiety comprises selective agents that are different within said targeting moiety, and the rest of targeting moieties comprise a single selective agent or selective agents that are the same within each of said targeting moieties. In yet another embodiment, the conjugate of the invention comprises more than one targeting moiety wherein the selective agents are single or different within each targeting moiety and wherein the targeting moieties comprising more than one selective agent comprise the same selective agents. In yet a further embodiment, the conjugate of the invention comprises more than one targeting moiety wherein the selective agents are single or different within each targeting moiety and wherein the targeting moieties comprising more than one selective agent comprise different combinations of selective agents.

In case the selectivity agents are different (within a targeting moiety of the invention or between targeting moieties of the conjugate of the invention) they are preferably all targeting specific proteins expressed by liver cells, even more preferably they are all targeting the same protein expressed by liver cells. Additionally, one or more of selectivity agents can have different binding specificity. A further particular embodiment refers to the conjugate of the invention wherein if the selectivity agents are different (within a targeting moiety of the invention or between targeting moieties of the conjugate of the invention), then they are all liver targeting moieties or they contain one or more selectivity agents having different binding specificity.

In a preferred embodiment, the selectivity agents are selected from the list consisting on: saccharides, polysaccharides, folate, biotin, retinoic acid, dehydroascorbic acid, antibodies, peptides, aptamers, transferrin, glycyrrhetinic acid, growth factors, lipoproteins, heat-labile enterotoxin subunit B, serotonin, carbohydrate polymers, arabinogalactan, pullulan, arabinogalactan, galactose, N-acetylgalactosamine, glucose and a combinations thereof.

In a preferred embodiment, at least one of the selectivity agents is a ligand of the asialoglycoprotein receptor, preferably a ligand selected from the list consisting on: carbohydrate polymers, arabinogalactan, pullulan, arabinogalactan, galactose, N-acetylgalactosamine, glucose and a combination thereof In a further preferred embodiment, the ligand of the asialoglycoprotein receptor is a compound of formula (A-1), (A-2), or (A-3): 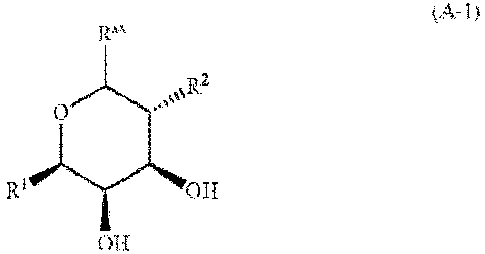 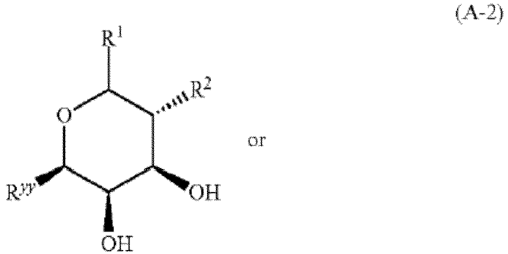 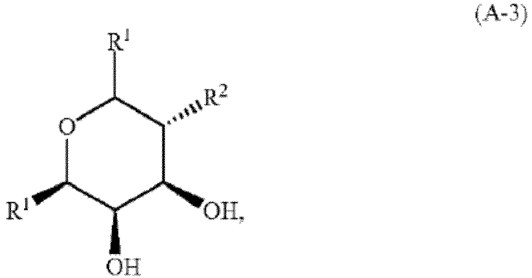 wherein:
R^{xx} is -H, -alkyl, -cycloalkyl, -alkenyl, alkynyl, -aryl, -heteroaryl, -OR⁵, -N(R⁴)-R⁵, - SR⁵, wherein a -CH₂- group of said R^{xx} may each be independently replaced with a heteroatom group selected from -O-,-S-, -N(R⁴)- and wherein a -CH₃ of said R^{xx} may be replaced with a heteroatom group selected from -N(R⁴)₂, -OR⁴, and -S(R⁴) wherein the heteroatom groups are separated by at least 2 carbon atoms, and wherein the alkyl, alkenyl, alkynyl, and cycloalkyl may each be substituted with one or more halo atoms;
R^{yy} is -CN, -CH₂-CN,-C≡CH, -CH₂-N₃,-CH₂-NH₂, -CH₂-N(R⁴)-S(O)₂-R⁵, -CH₂-CO₂H, -CO₂H, -CH₂-OH, -CH₂-SH, -CH=CH-R⁵, -CH₂-R⁵, -CH₂-S-R⁵, -CH₂-N(R⁴)-R⁵,-CH₂-N(R⁴)-C(O)-R⁵, -CH₂-N(R⁴)-C(O)-O-R⁵, -CH₂-N(R⁴)-C(O)-N(R⁴)-R⁵, -CH₂-O-R⁵, - CH₂-O-C(O)-R⁵, -CH₂-O-C(O)-N(R⁴)-R⁵, -CH₂-O-C(O)-O-R⁵, -CH₂-S(O)-R⁵, -CH₂-S(O)₂-R⁵, -CH₂-S(O)₂-N(R⁴)-R⁵, -C(O)-NH₂,-C(O)-O-R⁵, -C(O)-N(R⁴)-R⁵, or aryl or heteroaryl, wherein the aryl or heteroaryl is optionally substituted with R5;
each R¹ is independently -CN, -CH₂-CN, -C≡CH, -CH₂-N₃, -CH₂-NH₂, -CH₂-N (R₄)-S(O)₂-R⁵, -CH₂-CO₂H, -CO₂H, -CH₂-OH, -CH₂-SH, -CH=CH-R⁵, -CH₂-R⁵, -CH₂-S-R⁵, -CH₂-N(R⁴)-R⁵, -CH₂-N(R⁴)-C(O)-R⁵, -CH₂-N(R⁴)-C(O)-O-R⁵, -CH₂-N(R⁴)-C(O)-N(R⁴)-R⁵, -CH₂-O-R⁵, -CH₂-O-C(O)-R⁵, -CH₂-O-C(O)-N(R⁴)-R⁵, -CH₂-O-C(O)-O-R⁵, -CH₂-S(O)-R⁵, -CH₂-S(O)₂-R⁵, -CH₂-S(O)₂-N(R⁴)-R⁵, -C(O)-NH₂, -C(O)-O-R⁵, -C(O)-N(R⁴)-R⁵, or aryl or heteroaryl, wherein the aryl or heteroaryl is optionally substituted with R5,
or R¹ is -Z-X-Y, -Z-Y, -X-Y, or-Y;
X is a linker;
Y is the nucleic acid N of the conjugate of the invention;
Z is absent or is -C=C-, -CH=CH-, -CH₂-, -CH₂-O-, -C(O)-N(R⁴)-, -CH₂-S-, -CH₂-S(O)-, -CH₂-S(O)₂-,-CH₂-S(O)₂-N(R⁴)-, -C(O)-O-, -CH₂-N(R⁴)-, -CH₂-N(R⁴)-C(O)-, -CH₂-N(R⁴)-S(O)₂, -CH₂-N (R⁴)-C(O)-O-, -CH₂-N(R⁴)-C(O)-N(R⁴)-, -CH₂-O-C(O)-, -CH₂-O-C(O)-N(R⁴)-, -CH₂-O-C(O)-O-, or aryl or heteroaryl, wherein the aryl or heteroaryl is optionally substituted with R5;
R² is -OH, -N₃, -N(R³)₂, -N(R³)-C(O)R³, -N(R³)-C(O)-N(R³)₂, -N(R³)-C(O)-OR³, - N(R³)-S(O)₂-R³, tetrazole, or triazole, wherein the tetrazole and triazole are optionally substituted with R³;
each R³ is independently -H, -(C₁-C₅)alkyl, halo-substituted (C₁-C₅)alkyl, halo substituted (C₃-C₆) cycloalkyl, -(C₁-C₅)alkenyl, -(C₁-C₅)alkynyl, halo substituted -(C₁-C₅)alkenyl, halo substituted -(C₁-C₅) alkynyl, or (C₃-C₆)cycloalkyl, wherein a -CH₂-group of the alkyl or cycloalkyl may each be independently replaced with a heteroatom group selected from -O-, -S-, and -N(R⁴)- and -CH₃ of the alkyl may each be independently replaced with a heteroatom group selected from -N(R⁴)₂, -OR⁴, and - S(R⁴) wherein the heteroatom groups are separated by at least 2 carbon atoms;
each R⁴ is independently -H, -(C₁-C₂₀)alkyl, -(C₁-C₂₀)alkenyl, -(C₁-C₂₀)alkynyl, or (C₃-C₆)cycloalkyl wherein one to six -CH₂- groups of the alkyl or cycloalkyl separated by at least two carbon atoms may each be independently replaced with a heteroatom independently selected from -O-, -S-, or -N(R⁴)-, and -CH₃ of the alkyl may each be independently replaced with a heteroatom group selected from -N(R⁴)₂ , -OR⁴, and - S(R⁴) wherein the heteroatom groups are separated by at least 2 carbon atoms; and wherein the alkyl, alkenyl, alkynyl, and cycloalkyl may be substituted with halo atoms; each R⁵ is independently -H, (C₃-C₂₀)cycloalkyl, -(C₁-C₆₀)alkenyl, -(C₁-C₆₀)alkynyl, or (C₁-C₆₀)alkyl wherein one to six -CH₂ - groups of the cycloalkyl or one to 20 -CH₂-groups of the alkyl may each be independently replaced with heteroatoms independently selected from -O-, -S-, and -N(R⁴)- wherein the heteroatoms are separated by at least two carbon atoms, and -CH₃ of the alkyl may each be independently replaced with a heteroatom group selected from -N(R⁴)₂ , -OR⁴, and -S(R⁴) wherein the heteroatom groups are separated by at least 2 carbon atoms; and wherein the alkyl, alkenyl, alkynyl, and cycloalkyl may be substituted with halo atoms.

In another embodiment, the ligand of the asialoglycoprotein receptor is a compound with formula (B): 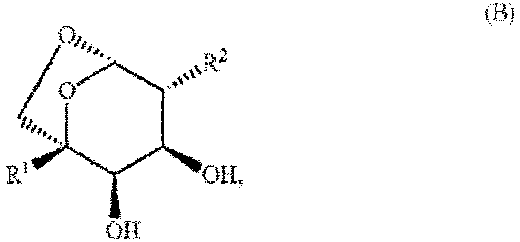 wherein:
R1 is -Z-X-Y, -Z-Y, -X-Y, or-Y;
X is a linker;
Y is the nucleic acid N of the conjugate of the invention;
Z is absent or is -C≡C-, --CH=CH-, -CH₂-, -CH₂-O-, -C(O)-N(R⁴)-, -CH₂-S-, -CH₂-S(O)-, -CH₂-S(O)₂-, -CH₂-S(O)₂-N(R⁴)-, -C(O)-O-, -CH₂-N(R⁴)-, -CH₂-N(R⁴)-C(O)-, -CH₂-N(R⁴)-S(O)₂-, -CH₂-N(R⁴)-C(O)-O-, -CH₂-N(R⁴)-C(O)-N(R⁴)-,-CH₂-O-C(O)-, -CH₂-O-C(O)-N(R⁴)-, -CH₂-O-C(O)-O-, or aryl or heteroaryl, wherein the aryl or heteroaryl is optionally substituted with R⁵;
R² is -OH, -N₃, -N(R³)₂ , -N(R³)-C(O)R³, -N(R³)-C(O)-N(R³)₂ , -N(R³)-C(O)-OR³, - N(R³)-S(O)₂-R³, tetrazole, or triazole, wherein the tetrazole and triazole are optionally substituted with R3;
each R³ is independently -H, -(C₁-C₅)alkyl, halo-substituted (C₁-C₅)alkyl, halo substituted (C₃-C₆) cycloalkyl, -(C₁-C₅)alkenyl, -(C₁-C₅)alkynyl, halo substituted -(C₁-C₅)alkenyl, halo substituted -(C₁-C₅) alkynyl, or (C₃-C₆)cycloalkyl, wherein a -CH₂-group of the alkyl or cycloalkyl may each be independently replaced with a heteroatom group selected from -O-, -S-, and -N(R⁴)- and -CH₃ of the alkyl may each be independently replaced with a heteroatom group selected from -N(R⁴)₂ , -OR⁴, and - S(R⁴) wherein the heteroatom groups are separated by at least 2 carbon atoms;
each R⁴ is independently -H, -(C₁-C₂₀)alkyl, -(C₁-C₂₀)alkenyl, -(C₁-C₂₀)alkynyl, or (C₃-C₆)cycloalkyl wherein one to six -CH₂ - groups of the alkyl or cycloalkyl separated by at least two carbon atoms may each be independently replaced with a heteroatom independently selected from -O-, -S-, or -N(R⁴)-, and -CH₃ of the alkyl may each be independently replaced with a heteroatom group selected from -N(R⁴)₂ , -OR⁴, and - S(R⁴) wherein the heteroatom groups are separated by at least 2 carbon atoms; and wherein the alkyl, alkenyl, alkynyl, and cycloalkyl may be substituted with halo atoms; each R⁵ is independently -H, (C₃-C₂₀)cycloalkyl, -(C₁-C₆₀)alkenyl, -(C₁-C₆₀)alkynyl, or (C₁-C₆₀)alkyl wherein one to six -CH₂ - groups of the cycloalkyl or one to 20 -CH₂ - groups of the alkyl may each be independently replaced with heteroatoms independently selected from -O-, -S-, and -N(R⁴)- wherein the heteroatoms are separated by at least two carbon atoms, and -CH₃ of the alkyl may each be independently replaced with a heteroatom group selected from -N(R⁴)₂ , -OR⁴, and -S(R⁴) wherein the heteroatom groups are separated by at least 2 carbon atoms; and wherein the alkyl, alkenyl, alkynyl, and cycloalkyl may be substituted with halo atoms.
In a more preferred embodiment, the ligand of the asialoglycoprotein receptor is the compound N-(1,3-bis[(1-{1-[(1S,2R,3R,4R,5S)-4-(acetylamino)-2,3-dihydroxy-6,8-dioxabicyclo[3.2.1]oct-1-yl]-2,5,8,11-tetraoxatridecan-13-yl}-1H-1,2,3-triazol-4-yl)methoxy]-2-{[(1-{1-[(1S,2R,3R,4R,5S)-4-(acetylamino)-2,3-dihydroxy-6,8-dioxabicyclo[3.2.1]oct-1-yl]-2,5,8,11-tetraoxatridecan-13-yl}-1H-1,2,3-triazol-4-yl)methoxy]methyl}propan-2-yl)-3,31-dioxo-1-(pyridin-2-yldisulfanyl) 7,10,13,16,19,22,25,28-octaoxa-4,32-diazaoctatriacontan-38-amide) with formula:

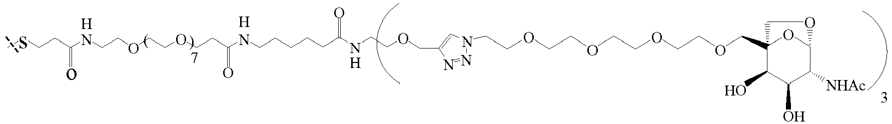

Regarding the ASGPR ligands, the patent application US 2017/0137801 A1 is is incorporated by reference herein.

In a more preferred embodiment the ligand of the asialoglycoprotein receptor comprises a GalNAc and/or a Galactose moiety. In a further preferred embodiment said ligand of the asialoglycoprotein receptor comprises a GalNAc moiety ((2*S*,3*R*,4*R*,5*R*,6*R*)-3-acetamido-4,5-dihydroxy-6-(hydroxymethyl)tetrahydro-2*H*-pyran-2-yl)oxy with formula:

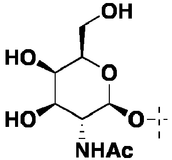

In a further embodiment the GalNAc moiety is a multiantennary GalNAc moiety, comprising at least two antennae, preferably at least 3 antennae, even more preferably at least 4 antennae. In a more preferred embodiment, the ligand of the asialoglycoprotein receptor is a triantennary GalNAc moiety 2,2,2-tris(3-((((6-(5-(((2*R*,3*R*,4*R*,5*R*,6*R*)-3-acetamido-4,5-dihydroxy-6-(hydroxymethyl)tetrahydro-2*H-*pyran-2-yl)oxy)pentanamido)hexyl)oxy)(hydroxy)phosphoryl)oxy)propoxy)methyl)ethoxy)etho xyl with formula:

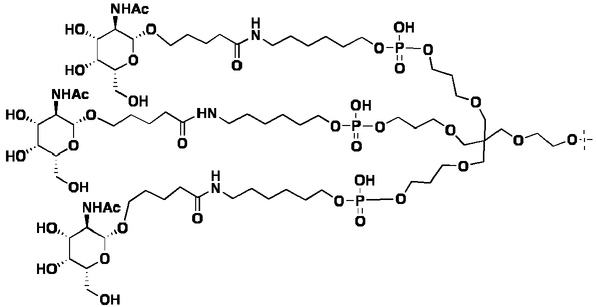

In a particular embodiment, the conjugate of the invention further comprises a group that facilitates the transport across biological membranes of the conjugate. Preferably, the group is amphipathic. An exemplary agents include, without limitation, no the signal sequence based peptide, PVEC, , amphiphilic model peptide, Arg9, bacterial cell wall permeating peptide, LL-37, cecropin PI, α-defensin, β-defensin, bactenectin, PR-39 and. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

In another particular embodiment of the invention, the conjugate of the invention further comprises an endosomolytic ligand. Endosomolytic ligands promote the lysis of the endosome and/or transport of the composition of the invention, or its components, from the endosome to the cytoplasm of the cell. The endosomolytic ligand may be a polyanionic peptide or peptidomimetic which shows pH-dependent membrane activity and fusogenicity. In certain embodiments, the endosomolytic ligand assumes its active conformation at endosomal pH. The "active" conformation is that conformation in which the endosomolytic ligand promotes lysis of the endosome and/or transport of the composition of the invention, or its components, from the endosome to the cytoplasm of the cell. Exemplary endosomolytic ligands include the GAL4 peptide (Subbarao et al., Biochemistry, 1987, 26: 2964-2972), the EALA peptide (Vogel et al., J. Am. Chem. Soc., 1996, 118: 1581-1586), and their derivatives (Turk et al., Biochem. Biophys. Acta, 2002, 1559: 56-68), the INF-7 peptide, the diINF-7 peptide, the diINF3 peptide, the GLF peptide, the GALA-INF3 peptide, the INF-5 peptide, In certain embodiments, the endosomolytic component may contain a chemical group (e.g., an amino acid) which will undergo a change in charge or protonation in response to a change in pH. The endosomolytic component may be linear or branched.

In a further particular embodiment of the invention, the endosomolytic ligands for use in the present invention include, but are not limited to, a lysosomotropic agent, a cell penetrating peptide, a fusogenic peptide, an endosomolytic peptide, a pore forming agent, and a proton sponge agent.

Lysosomotropic agents are weak bases that can penetrate in lysosome as protonated form and increase the intracellular pH. Suitable lysosomotropic agents for use in the present application include, but are not limited to chlorpromazine, amantadine, 4-aminoquinoline, amiodarone, amodiaquine, azithromycin, chloroquine, clindamycin, N-(3-[(2,4-dinitropheny1)-amino]-propyl)-N-(3-aminopropyl-methylamine) dihydrochloride (DAMP), imipramine, methylamine, monensin, monodansylcadaverine, NH₄Cl, perhexilene, phenylalanine methyl ester, primaquine, quinacrine, suramin, thioridazine, tilorone, tributylamine, ketotifen fumarate, glycerol, and sucrose. In some embodiments, the lysosomotropic agent is chloroquine, glycerol or sucrose.

Cell penetrating peptides (CPPs) are a class of diverse peptides, typically with 5-30 amino acids, that can cross the cellular membrane. In some embodiments, suitable CPPs for use herein include cationic CPPs, amphipathic CPPs, and hydrophobic CPPs. Suitable CPPs for use herein include, but are not limited to, CPPs derived from heparin-, RNA-, and DNA-binding proteins such as those consisting on SEQ ID no.: 21-50, CPPs derived from signal peptides such as those consisting on SEQ ID no.:51-54, CPPs derived from antimicrobial peptides such as those consisting on SEQ ID no.: 55-65, CPPs derived from viral proteins such as those consisting on SEQ ID no.: 66-72, CPPs derived from various natural proteins such as those consisting on SEQ ID no.:73-87, and designed CPPs and CPPs derived from peptide libraries such as those consisting on SEQ ID no.:88-136. See, e.g., Milletti, F. Drug Discovery Today, Volume 17, Numbers 15/16, 2012, 850-860.

Fusogenic peptides are short peptides that destabilize the phospholipid membrane. In some embodiments, a fusogenic peptide comprises 20-30 amino acids. They primarily elicit endosomal escape by two main mechanisms: membrane fusion and pore formation. Suitable fusogenic peptides for use herein include, but are not limited to, an influenza HA-2 peptide, GLFGAIAGFIENGWEGMIDGWYG (SEQ ID no: 137); melittin, GIGAVLKVLTTGLPALISWIKRKRQQ (SEQ ID no: 138); tat (48-60), GRKKRRQRRRPPQ (SEQ ID no: 139); penetratin, RQIKIWFQNRRMKWKK (SEQ ID no: 140); transportan, GWTLNSAGYLLGKINLKALAALAKKIL (SEQ ID no: 141); GALA peptide, WEAALAEALAEALAEHLAEALAEALEALAA (SEQ ID no: 142); KALA peptide, WEAKLAKALAKALAKHLAKALAKALKACEA (SEQ ID no: 143); JST-1, GLFEALLELLESLWELLLEA (SEQ ID no: 144); ppTGl, GLFKALLKLLKSLWKLLLKA (SEQ ID no: 145); ppTG20, GLFRALLRLLRSLWRLLLRA (SEQ ID no: 146); and derivatives thereof. In some embodiments, the endosomolytic ligand comprises a KALA peptide, WEAKLAKALAKALAKHLAKALAKALKACEA (SEQ ID no: 143).

In some embodiments, the endosomolytic ligand is an endosomolytic histidine-rich peptide selected from, but not limited to, CHK₆HC (SEQ ID NO:147); H5WYG (SEQ ID no: 148), GLFHAIAHFIHGGWHGLIHGWYG (SEQ ID N0:149); and derivatives thereof. In some embodiments, the endosomolytic ligand comprises a HSWYG (SEQ ID no: 148), or GLFHAIAHFIHGGWHGLIHGWYG (SEQ ID N0:149) peptide. In some embodiments, the endosomolytic ligand is a synthetic peptide. In some embodiments, the endosomolytic ligand is peptide ppTG21: GLFHALLHLLHSLWHLLLHA (SEQ ID no: 150), having the structure: 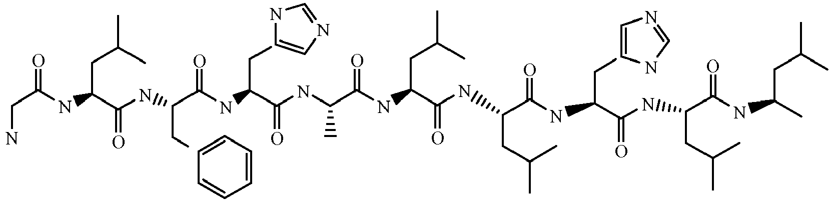 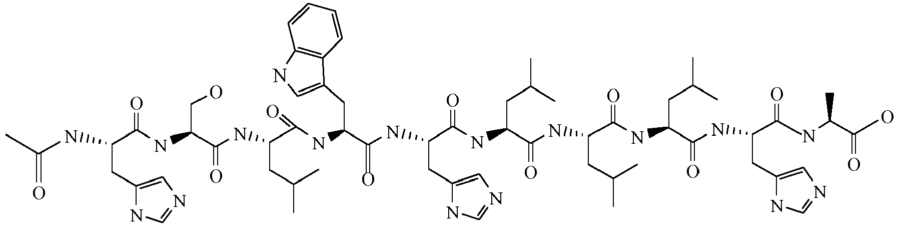 wherein the hydrogen atoms are omitted from the peptide structure for clarity of view. See Rittner, Karola, et al., New Basic Membrane-Destabilizing Peptides for Plasmid-Based Gene Delivery in Vitro and in Vivo, MOLECULAR THERAPY 5(2) 104-114 (2002).

Pore forming agents are agents, such as peptides, that induce pore formation through the membrane thereby disrupting endosome. Suitable pore-forming agents for use herein include, but are not limited to, cecropin (insects), magainin, CPF 1, PGLa, Bombinin BLP-1 (all three from amphibians), melittin (bees), seminalplasmin (bovine), indolicidin, bactenecin (both from bovine neutrophils), tachyplesin 1 (crabs), protegrin (porcine leukocytes), and defensins (from human, rabbit, bovine, fungi, and plants), Gramicidin A and gramicidin S (*bacillus brevis*), the lantibiotics such as nisin (*lactococcus lactis*), androctonin (scorpion), cardiotoxin I (cobra), caerin (frog litoria splendida), dermaseptin (frog). Viral peptides have also been shown to have pore-forming activity, examples include hemagglutinin subunit HA-2 (influenza virus), E1 (Semliki forest virus), F1 (Sendai and measles viruses), gp41 (HIV), gp32 (SIV), and vp1 (Rhino, polio, and coxsackie viruses). In some embodiments, the endosomal escape gent is a vpl peptide derived from rhino virus. In some embodiments, the endosomolytic ligand is the mellitin peptide derived from bee venom, or a masked analog thereof.

Proton sponge agents are agents having multiple proton acceptor sites that disrupt the endosome by osmolytic action. In some embodiments, the proton sponge agent suitable for use herein comprises a plurality of proton acceptor sites having pKa values between physiological and lysosomal pH. In some embodiments, the proton sponge agent suitable for use herein comprises a plurality of proton acceptor sites having pKa values within the range of 4 to 7, which endosomal lysing component is polycationic at pH 4. In some embodiments, the endosomolytic ligand is a proton sponge agent selected from imidazole-containing compounds such as compounds or peptides comprising one or more histidine, histamine, vinylimidazole, or combinations thereof. In some embodiments, the suitable proton sponge agents for use herein include polymers that have one or more secondary or tertiary amines and exhibit pKa values between physiological and lysosomal pH. In some embodiments, the proton sponge agents suitable for use herein include, but are not limited to polymers such as plyethylenimines, plyamidoamine dendrimers, PEG-oligo(glutamic acid)-PEI; poly(L-histidines); chloroquine, methylamine, ammonium chloride.
In a further embodiment of the invention, the conjugate further comprises nuclear localization sequence (NLS). "Nuclear localization sequence" (NLS) refers to an amino acid sequence which assists the conjugate of the present invention to enter the nucleus of a eukaryotic cell. Consequently, an NLS typically comprises one or more short sequences of positively charged lysines or arginines exposed on the protein surface. Exemplary NLSs include, but are not limited to, an NLS sequence derived from: the NLS of the SV 40 virus large T-antigen, having the amino acid sequence PKKKRKV (SEQ ID no: 151); the NLS from nucleoplasmin (e.g. the nucleoplasmin bipartite NLS with the sequence KRPAATKKAGQAKKKK (SEQ ID no: 152); the c-myc NLS having the amino acid sequence PAAKRVKLD (SEQ ID no: 153) or RQRRNELKRSP (SEQ ID no: 154); the hRNPA1 M9 NLS having the sequence NQSSNFGPMKGGNFGGRSSGPYGGGGQYFAKPRNQGGY (SEQ ID no: 155); the sequence RMRIXFKNKGKDTAELRRRRVEVSVELRKAKKDEQILKRRNV (wherein X is any amino acid) (SEQ ID no: 156) of the IBB domain from importinalpha; the sequences VSRKRPRP (SEQ ID no: 157) and PPKKARED (SEQ ID no: 158) of the myoma T protein; the sequence PQPKKKPL (SEQ ID no: 159) of human p53; the sequence SALIKKKKKMAP (SEQ ID no: 160) of mouse c-abl IV; the sequences DRLRR (SEQ ID no: 161) and PKQKKRK (SEQ ID no: 162) of the influenza virus NS1; the sequence RKLKKKIKKL (SEQ ID no: 163) of the Hepatitis virus delta antigen; the sequence REKKKFLKRR (SEQ ID no: 164) of the mouse Mx1 protein; the sequence KRKGDEVDGVDEVAKKKSKK (SEQ ID no: 165) of the human poly(ADP-ribose) polymerase; the sequence RKCLQAGMNLEARKTKK (SEQ ID no: 166) of the steroid hormone receptors (human) glucocorticoid; the sequence MAPKKKRKVGIHRGVP (SEQ ID N0:167); and the sequence PKKKRKVEDPKKKRKVD (SEQ ID N0:168).

Regarding the endosomolytic ligands and the NLS, the patent application US 2017/0137801 A1 is introduced herein as reference.

### A.2.3 Linker regions of the conjugates of the invention

The nucleic acid N and the targeting moiety or moieties of the conjugate of the invention may be directly coupled. However, in an embodiment of the invention both moieties are linked by a connecting group.

The terms "connecting group", "linker", "linking group" and grammatical equivalents thereof are used herein to refer to an organic moiety that connects two parts of a conjugate. The targeting moiety can be linked to any nucleotide within the nucleic acid of the invention, thus it can be linked through an internal nucleotide or through the 3' terminal nucleotide and/or 5' terminal nucleotide. An internal conjugate may be attached directly or indirectly through a linker to a nucleotide at a 2' position of the ribose group, or to another suitable position.

In the case wherein the nucleic acid is a double-stranded nucleic acid, the targeting moiety can be linked to the sense 3' terminal nucleotide, the sense 5' terminal nucleotide, the antisense 3' terminal nucleotide, and/or the antisense 5' terminal nucleotide.

Though not wishing to be limited by definitions or conventions, in this application the length of the linker is described by counting the number atoms that represent the shortest distance between the atom that joins the conjugate moiety to the linker and the oxygen atom of the terminal phosphate moiety associated with the oligonucleotide through which the linker is attached to the oligonucleotide. In cases where the linker comprises one or more ring structures, counting the atoms around the ring that represent the shortest path is preferred.

Suitable linker groups for use in the present invention include, without limitation, modified or unmodified nucleotides, nucleosides, polymers, sugars, carbohydrates, polyalkylenes such as polyethylene glycols and polypropylene glycols, polyalcohols, polypropylenes, mixtures of ethylene and propylene glycols, polyalkylamines, polyamines such as polylysin and spermidine, polyesters such as poly(ethyl acrylate), polyphosphodiesters, aliphatics, and alkylenes. Moreover, linkers/linker chemistries that are based on omega-amino-1,3- diols, omega-amino-1,2-diols, hydroxyprolinols, omega-amino-alkanols, diethanolamines, omega- hydroxy-1,3-diols, omega-hydroxy-1,2-diols, omega-thio-1,3-diols, omega-thio -1,2-diols, omega-carboxy-1,3-diols, omega-carboxy -1,2-diols, co-hydroxy-alkanols, omega-thio-alkanols, omega- carboxy-alkanols, functionalized oligoethylene glycols, allyl amine, acrylic acid, allyl alcohol, propargyl amine, propargyl alcohol, and more, can be applied in this context to generate linkers of the appropriate length.

The linker may also confer other desirable properties on the oligonucleotide conjugate such as improved aqueous solubility, optimal distance of separation between the targeting moiety and the oligonucleotide, flexibility (or lack thereof), specific orientation, branching, and others.

In a particular embodiment, the composition of the invention may be used for the production of a non-human transgenic animal or transgenic plant, provided that the use is not a method for treatment of the animal body by therapy.

### A.3. The complex of the invention formed between the mRNA of interest and the nucleic acid N of the conjugate of the invention

As indicated previously (section A.2.1), the nucleic acid of the conjugate of the invention and the mRNA of interest are at least substantially complementary and form the complex of the invention. The preferred lengths of the sequences within the nucleic acid N and within the mRNA of the complex involved in the base pairing are also indicated therein (section A.2.1).

The region in the mRNA of interest involved in the base-pairing with the nucleic acid N can be different from the poly(A) tail. Nonetheless, in a preferred embodiment, the nucleic acid N of the conjugate of the invention comprises a poly-uridine or a poly-thymidine region that can base pair with or bind to the poly(A) tail of the mRNA of the complex of the invention. Accordingly, a preferred embodiment refers to the complex of the invention wherein the nucleic acid N which forms part of the conjugate of the invention comprises a poly-uridine or poly-thymidine region and wherein the complex is formed by base pairing between said poly-uridine or poly-thimidine region and the poly(A) tail within the mRNA. In a preferred embodiment, the nucleic acid N of the conjugate of the invention comprises a poly-uridine region that base-pairs with a region of the poly(A) tail of the mRNA of the complex of the invention, wherein the number of uridine nucleotides comprised in the poly-uridine region of the nucleic acid N varies from at least 5, at least 10, at least 15, at least 20, at least 25, at least 30, at least 35, to less than 100, less than 150, less than 200, less than 250. More preferably, the poly-uridine region of the nucleic acid N is of at least 15 nt. In another preferred embodiment, the nucleic acid N of the conjugate of the invention comprises a poly-thymidine region that base-pairs with a region of the poly(A) tail of the mRNA of the complex of the invention, wherein the number of thymidine nucleotides comprised in the poly-thymidine region of the nucleic acid N varies from at least 5, at least 10, at least 15, at least 20, at least 25, at least 30, at least 35, to less than 100, less than 150, less than 200, less than 250. More preferably, the poly-thymidine region of the nucleic acid N is of at least 15 nt.

In another embodiment, the nucleic acid of the conjugate of the invention interacts with a region of the mRNA of the complex of the invention that is not the poly(A) tail and wherein the complex is formed by base pairing between a sequence comprised in the nucleic acid of the conjugate of the invention and said region of the mRNA different from the poly(A) tail. The length of the sequence of the nucleic acid that base pairs with a region of the mRNA different from the poly(A) tail of the mRNA of the complex of the invention is selected from those indicated in section A.2.1.

In a further embodiment, the nucleic acid of the conjugate of the invention interacts with a region of the poly(A) tail of the mRNA of the complex of the invention as well as with a region of the mRNA that is different from the poly(A) tail, wherein the complex is formed by base pairing between sequences comprised in the nucleic acid of the conjugate of the invention and said regions of the mRNA. The nucleic acid N of the conjugate of the invention thus comprises a poly-uridine or a poly-thymidine region, wherein the corresponding number of uridines or thymidines varies from at least 5, at least 10, at least 15, at least 20, at least 25, at least 30, at least 35, to less than 100, less than 150, less than 200, less than 245 and a sequence that base pairs with a region of the mRNA different from the poly(A) tail with a length that varies from at least 5 nt, at least 10 nt, at least 15 nt, at least 20 nt, at least 25 nt, at least 30 nt, at least 35 nt, to less than 100 nt, less than 150 nt, less than 200 nt, less than 245 nt. Similarly, the length of the sequence from the mRNA region involved in the base pairing with the nucleic acid of the conjugate of the invention, including a region from the poly(A) tail and a region different from the poly(A) tail, can vary from at least 5 nt, at least 10 nt, at least 15 nt, at least 20 nt, at least 25 nt, at least 30 nt, at least 35 nt, to less than 100 nt, less than 150 nt, less than 200 nt, less than 250 nt.

In a further embodiment, several nucleic acids of the invention being each one part of a different conjugate of the invention bind to different regions within the mRNA of the invention. In other words, the composition of the invention may comprise a plurality of conjugates as defined in the invention, wherein the nucleic acids within each conjugate bind to different regions within the mRNA. Said regions within the mRNA can be different from the poly(A) tail. Nonetheless, in a preferred embodiment, the composition of the invention may comprise a plurality of nucleic acids being each one part of a different conjugate of the invention, wherein the nucleic acid of at least one of the conjugates of the invention interacts with the poly(A) tail of the mRNA of the invention. Thus, a preferred embodiment refers to the complex of the invention wherein the nucleic acid which forms part of at least one of the conjugates comprises a poly-uridine or poly-thymidine region and wherein the complex is formed by base pairing between said poly-uridine or poly-thymidine region and the poly(A) tail within the mRNA. Preferably, the length of the poly-uridine region and the length of the poly-thymidine region are those indicated as preferred ones in the segond paragraph of this section A.3. A further embodiment refers to the complex of the invention wherein the complex comprises a first conjugate, wherein the nucleic acid within said first conjugate comprises a poly-uridine or poly-thymidine region and at least a second conjugate wherein the nucleic acid has a sequence which is substantially complementary to a region of the mRNA which is not the poly(A) region wherein a complex is formed between the nucleic acid of the first conjugate by base pairing between said polyuridine or poly-thymidine and the poly(A) tail within the mRNA and wherein a complex is formed by base pairing between the nucleic acid within the second conjugate and the region within the mRNA which is not the poly(A) tail within the mRNA. In other words, in an embodiment, the nucleic acid of at least one of the conjugates of the invention interacts with the poly(A) tail of the mRNA of the invention, and the nucleic acid of at least one additional conjugate interacts with a region of the mRNA of the invention that is not the poly(A) tail. Preferably, the length of the sequence of the nucleic acid of the first conjugate that base pairs with the poly(A) tail of the mRNA of interest is selected from those indicated as preferred ones in the previous paragraph; and the length of the sequence of the nucleic acid of the second conjugate that base pairs with a region of the mRNA different from the poly(A) tail is selected from those indicated in section A.2.1.

A further preferred embodiment refers to a complex of the invention wherein the nucleic acids of all the conjugates of the invention comprise a poly-uridine or poly-thymidine region and wherein the complex is formed by base pairing between said poly-uridine or poly-thymidine regions and the poly(A) tail within the mRNA. Preferably, the length of the sequence of the nucleic acids that base pair with the poly(A) tail of the mRNA of interest is selected from those indicated as preferred ones in the second paragraph of this section A3;

In another embodiment, the composition of the invention comprises a plurality of nucleic acids being each one part of a different conjugate of the invention, wherein the nucleic acid of each conjugate of the invention interacts with a region of the mRNA of the complex of the invention that is not the poly(A) tail and wherein the complex is formed by base pairing between a sequence comprised in the nucleic acid of the conjugate of the invention and said region of the mRNA that is different from the poly(A) tail. The length of the sequence of the nucleic acid that base pairs with a region of the mRNA different from the poly(A) tail is selected from those indicated in section A.2.1. Similarly, the length of the region of the mRNA that base pairs with the nucleic acid of each nucleic is selected from the list selected from those indicated in section A.2.1.

In a further embodiment, the composition of the invention comprises a plurality of nucleic acids being each one part of a different conjugate of the invention, wherein the nucleic acid of at least one conjugate of the invention interacts with a region of the poly(A) tail of the mRNA of the complex of the invention as well as with a region of the mRNA that is different from the poly(A) tail, wherein the complex is formed by base pairing between sequences comprised in the nucleic acid of the conjugate of the invention and said regions of the mRNA. Thus, in a preferred embodiment, the nucleic acid N of at least one conjugate of the invention comprises a poly-uridine or a poly-thymidine region that base pairs with a region of the poly(A) tail of the mRNA, wherein the corresponding number of uridines or thymidines varies from at least 5, at least 10, at least 15, at least 20, at least 25, at least 30, at least 35, to less than 100, less than 150, less than 200, less than 245 and a sequence that base pairs with a region of the mRNA different from the poly(A) tail with a length that varies from at least 5 nt, at least 10 nt, at least 15 nt, at least 20 nt, at least 25 nt, at least 30 nt, at least 35 nt, to less than 100 nt, less than 150 nt, less than 200 nt, less than 245 nt. Similarly, the length of the sequence from the mRNA region involved in the base pairing with sthe nucleic acid of said conjugate of the invention, including a region from the poly(A) tail and a region different from the poly(A) tail, can vary from at least 5 nt, at least 10 nt, at least 15 nt, at least 20 nt, at least 25 nt, at least 30 nt, at least 35 nt, to less than 100 nt, less than 150 nt, less than 200 nt, less than 250 nt.

In a preferred embodiment, the complementary region between the nucleic acid N of the conjugate of the invention and the mRNA of the complex of the invention comprises non-canonical base pairs, such as Hoogsteen base pairs. "Hoogsteen base pairs" refer to hydrogen bonds between the side of the purine base that faces the major groove and the Watson-Crick base pairing face of the pyrimidine. Hydrogen bonds in the Hoogsteen base pair are formed between the purine N7 to the pyrimidine N3 and either the adenine N6 to the thymine O6 or the guanine O4 to the cytidine N4. Said Hoogsteen base pairs may facilitate the recognition of the complementary region by a specific protein and/or the formation of triple or quadruple base pairs.

In another preferred embodiment, the complementary region formed by base pairing between the nucleic acid and the mRNA of the invention further comprises one or more interstrand covalent bonds between nucleotides of the nucleic acid and the mRNA of the invention. "Interstrand covalent bond" means that a nucleotide from one strand of the complex (from the nucleic acid of the invention or from the mRNA of the invention) is covalently joined to a nucleotide from the opposite strand of the complex (the mRNA of the invention if the other strand was the nucleic acid of the invention and vis versa). The nucleotides covalently bound thereof are directly covalently joined between each other, or indirectly covalently joined between each other through an intervening moiety or moieties, such as a linker, or a bridge, or a spacer, moiety or moieties.

The covalent bond may be mediated by furocoumarins, preferably by psoralen. Thus, in a preferred embodiment, the covalent bonds referred above, comprised in the complementary region formed by base pairing between the nucleic acid of the invention and the mRNA of interest, are formed by psoralen cross-link.

Furocoumarins are tricyclic aromatic compounds intercalate into DNA and form covalent adducts with pyrimidine bases when exposed to near ultraviolet light (UVA). Psoralens constitute a subclass of molecules having a linear arrangement of the tricyclic rings. Bifunctional psoralens contain two reactive sites, the 3,4-pyrone and the 4',5'-furan double bonds, either of which, when activated by UVA, reacts specifically with the 5,6-double bond of pyrimidine bases, forming cyclobutane-type monoadducts. Upon further irradiation, a fraction of the 4',5'-furan side monoadducts (MAfs) photoreacts with a pyrimidine base on the opposite DNA strand to form an interstrand cross-link. The ratio of monoadducts (MAs) over cross-links formed may vary according to the structural nature of the bifunctional psoralens and to the dose of UVA (generally 365 nm) used for photoactivation. The fraction of MAs is, however, generally higher than that of cross-links. It has been reported that in vivo or in vitro irradiation at 405 nm of DNA containing bifunctional psoralens leads to the production of only MAs (Tessman et al. 1985; Averbeck et al. 1987), whereas treatment at 365 nm induces both MAs and cross-links (Moustacchi E., The Target Organ and the Toxic Process; Arch. Toxicol., 1988 (Suppl. 12) 26-34).
In a further preferred embodiment, the psolaren moiety 6-[4'-(Hydroxymethyl)-4,5',8-trimethylpsoralen]-hexyl with the formula: 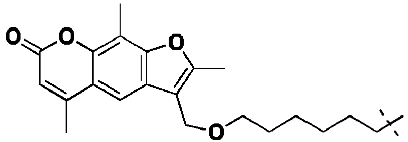 is covalently linked to the 5' and/or the psoralen moiety 6-[4'-(Hydroxymethyl)-4,5',8-trimethylpsoralen]-2 methoxy propyl with the formula: 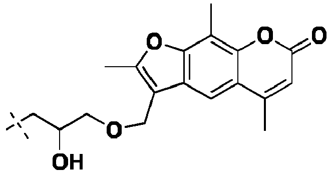 is covalently linked to the 3' end of the Ligand conjugated sequence and forms a cross-link with a pyrimidine nucleotide of the mRNA of the complex.

A non-limiting example of a psolaren moiety covalently linked to a nucleic acid could be a psoralen derivative 4, 5', 8-trimethylpsoralen covalently linked to the 5' end of the nucleic acid of the invention. Said psolaren derivative could be covalently linked to the 5'-terminus of the nucleic acid in the course of solid phase synthesis using phosphoroamidite chemistry. In this case, the derivative could be introduced as a phosphitylation compound in the last cycle of the oligomer synthesis. nonoAfter oxydation and deprotection the 5'-psoralen modified nucleic acid can be purified by HPLC (High pressure liquid chromatography) and characterised by Maldi-Tof mass spectrometry. (matrix-assisted laser desorption/ionization Time of flight). Hybridisation of the psoralen-modified nucleic acid to a substantially complementary polynucleotide sequence would be followed by irradiation at 350 nm to induce covalent cross-link between the psolaren-modifed nucleic acid and the substantially complementary polynucleotide sequence. The cross-link could be reversed upon irradiation at 254nm. (Pieles U. and Englisch U., Nucleic Acids Res. 1989 Jan 11; 17(1): 285-299).

In a preferred embodiment of the invention, the mRNA forming part of the complex of the invention further comprises one or more components that increase the stability of the complex while maintaining its targeting specificity, such as . Examples of said compositions include a cationic or polycationic a cationic or polycationic compound, suitable for complexing and thereby stabilizing the mRNA. Particularly preferred, cationic or polycationic peptides or proteins may be selected from protamine, nucleoline, spermine or spermidine, poly-L-lysine (PLL), basic polypeptides, poly-arginine, proline-rich peptides, arginine-rich peptides, lysine-rich peptides, Pep-1, Calcitonin peptide(s), etc.

In a preferred embodiment, the additional component favoring stabilization of the mRNA is spermine or protamine.

The term "protamine" as used herein, refers to a low molecular weight cationic, arginine-rich polypeptide. The protamine molecule typically comprises about 20 to about 200 amino acids and is generally characterized by containing at least 20 percent, 50 percent or 70 percent arginine. As used herein, the term "spermine" refers to a compound having the CAS number 71-44-3.

In another preferred embodiment, the complex of the invention is complexed with protamines. In a further preferred embodiment, the complex of the invention is complexed with spermine polymers.

In another embodiment, the complexes of the invention are found within higher order structures that increase the stability of the complex while maintaining its targeting specificity. Examples of said structures include, without limitation, micelles, liposomes, cationic liposomes, lipid nanoparticles (LNPs).

In one embodiment, the complex is incorporated in a liposome thereby forming a lipid nanoparticle or LNP. The liposome-incorporated complexes can be completely or be partially located in the interior space of the liposome, within the bilayer membrane of the liposome, or associated with the exterior surface of the liposome membrane. The incorporation of a complex or of the mRNA into liposomes is referred to herein as "encapsulation," wherein the complex or the mRNA is entirely contained within the interior space of the liposome. The purpose of incorporating an mRNA into a transfer vehicle, such as a liposome, is often to protect the nucleic acid from an environment that may contain enzymes or chemicals that degrade nucleic acids and/or systems or receptors that cause the rapid excretion of the nucleic acids. Accordingly, the selected transfer vehicle is capable of enhancing the stability of the mRNA contained therein. The liposome allows the encapsulated mRNA to reach a desired target cell.

Suitable lipids that can be used to form a liposome include any member of a group of organic compounds that has lipophilic or amphipathic properties, including, but not limited to, fats, fatty oils, essential oils, waxes, steroids, sterols, phospholipids, glycolipids, sulpholipids, aminolipids, chromolipids (lipochromes), and fatty acids. The term "lipid" encompasses both naturally occurring and synthetically produced lipids. Particular lipids include DOTAP, DOPS and cholesterol. Lipids can include cationic lipids. As used herein, the term "cationic lipid" encompasses any of a number of lipid species that carry a net positive charge at physiological pH, which can be determined using any method known to one of skill in the art. These include, but are not limited to, N-methyl-N- (2-(arginoylamino) ethyl)-N, N- Di octadecyl aminium chloride or di stearoyl arginyl ammonium chloride] (DSAA), N,N-di-myristoyl-N-methyl-N-2[N'-(N6-guanidino-L- lysinyl)] aminoethyl ammonium chloride (DMGLA), N,N-dimyristoyl-N-methyl-N-2[N2- guanidino-L-lysinyl] aminoethyl ammonium chloride, N,N-dimyristoyl-N-methyl-N-2[N'- (N2, N6-di-guanidino-L-lysinyl)] aminoethyl ammonium chloride, and N,N-di-stearoyl-N-methyl-N-2[N'-(N6-guanidino-L-lysinyl)] aminoethyl ammonium chloride (DSGLA). Other non-limiting examples of cationic lipids that can be present include N,N-dioleyl-N,N-dimethylammonium chloride ("DODAC"); N-(2,3-dioleoyloxy) propyl)-N,N,N-trimethylammonium chloride ("DOTAP"); N-(2,3-dioleyloxy) propyl)- -trimethylammonium chloride ("DOTMA") or other N-(N,N-1 -dialkoxy)-alkyl- -trisubstituted ammonium surfactants; N,N-distearyl-N,N-dimethylammonium bromide ("DDAB"); 3-(N-(N',N'-dimethylaminoethane)-carbamoyl) cholesterol ("DC- Chol") and N-(1,2-dimyristyloxyprop-3-yl)-N,N-dimethyl-N-hydroxyethyl ammonium bromide ("DMRIE"); 1,3-dioleoyl-3-trimethylammonium-propane, N-(1-(2,3- dioleyloxy)propyl)-N-(2-(sperminecarboxamido)ethyl)-N,N-dimethy- 1 ammonium trifluoro-acetate (DOSPA); GAP-DLRIE; DMDHP; 3-p[4N-('N,8N-diguanidino spermidine)-carbamoyl] cholesterol (BGSC); 3-P[N,N-diguanidinoethyl-aminoethane)- carbamoyl] cholesterol (BGTC); N,N',N2,N3 Tetra-methyltetrapalmitylspermine (cellfectin); N-t-butyl-N'-tetradecyl-3-tetradecyl-aminopropion-amidine (CLONfectin); dimethyldioctadecyl ammonium bromide (DDAB); 1,3-dioleoyloxy-2-(6-carboxyspermyl)- propyl amide (DOSPER); 4-(2,3-bis-palmitoyloxy-propyl)-1-methyl- 1 H-imidazole (DPIM) ?,?,?',?'-tetramethy 1-N,N'-bis(2-hydroxyethyl)-2,3 dioleoyloxy- 1,4-butanediammonium iodide) (Tfx-50); 1,2 dioleoyl-3-(4'-trimethylammonio) butanol-sn-glycerol (DOBT) or cholesteryl (4'trimethylammonia) butanoate (ChOTB) where the trimethylammonium group is connected via a butanol spacer arm to either the double chain (for DOTB) or cholesteryl group (for ChOTB); DL- 1,2-dioleoyl-3-dimethylaminopropyl-P-hydroxyethylammonium (DORI) or DL-1,2-0-dioleoyl-3-dimethylaminopropyl-P-hydroxyethylammonium (DORIE) or analogs thereof as disclosed in International Application Publication no. WO 93/03709, which is herein incorporated by reference in its entirety; 1,2-dioleoyl-3-succinyl- sn-glycerol choline ester (DOSC); cholesteryl hemisuccinate ester (ChOSC); lipopolyamines such as dioctadecylamidoglycylspermine (DOGS) and dipalmitoyl phosphatidylethanolamylspermine (DPPES) or the cationic lipids disclosed in U.S. Pat. no. 5,283, 185, which is herein incorporated by reference in its entirety; cholesteryl-3p- carboxyl-amido-ethylenetrimethylammonium iodide; 1 - dimethylamino-3- trimethylammonio-DL-2-propyl-cholesteryl carboxylate iodide; cholesteryl-3- beta - carboxyamidoethyleneamine; cholesteryl-3-p-oxysuccinamido-ethylenetrimethylammonium iodide; 1 -dimethylamino-3-trimethylammonio-DL-2-propyl- cholesteryl-3-P-oxysuccinate iodide; 2-(2-trimethylammonio)-ethylmethylamino ethyl- cholesteryl-3-P-oxysuccinate iodide; and 3-P-N-(polyethyleneimine)- carbamoylcholesterol.

The lipids can contain co-lipids that are negatively charged or neutral. As used herein, a "co-lipid" refers to a non-cationic lipid, which includes neutral (uncharged) or anionic lipids. The term "neutral lipid" refers to any of a number of lipid species that exist either in an uncharged or neutral zwitterionic form at physiological pH. The term "anionic lipid" encompasses any of a number of lipid species that carry a net negative charge at physiological pH. Co-lipids can include, but are not limited to, diacylphosphatidylcholine, diacylphosphatidylethanolamine, ceramide, sphingomyelin, cephalin, cholesterol, cerebrosides and diacylglycerols, phospholipid-related materials, such as lecithin, phosphatidylethanolamine, lyso lecithin, lysophosphatidylethanolamine, phosphatidylserine, phosphatidylinositol, cardiolipin, phosphatidic acid, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), palmitoyloleyolphosphatidylglycerol (POPG), dipalmitoylphosphatidylglycerol (DPPG), dioleoyl-phosphatidylethanolamine (DOPE), palmitoyloleoylphosphatidylchol- ine (POPC), palmitoyloleoylphosphatidylethanolamine (POPE), dioleoyl- phosphatidylethanolamine 4-(N-maleimidomethyl)-cyclohexane-1-carboxylate (DOPE- mal), dioleoyl phosphatidic acid (DOPA), stearylamine, dodecylamine, hexadecylamine, acetyl palmitate, glycerolricinoleate, hexadecyl stereate, isopropyl myristate, amphoteric acrylic polymers, triethanolamine-lauryl sulfate, alkyl-aryl sulfate polyethyloxylated fatty acid amides, lysophosphatidylcholine, and dioctadecyldimethyl ammonium bromide and the like. Co-lipids also include polyethylene glycol-based polymers such as PEG 2000, PEG 5000 and polyethylene glycol conjugated to phospholipids or to ceramides, as described in U.S. Pat. no. 5,820,873, herein incorporated by reference in its entirety.

Preferably, the amphiphilic lipid having a free phosphate group is dioleoyl phosphatidic acid (DOPA).

The surface of the nanoparticles can be PEGylated. The term "polymer-PEG conjugate" also refers to these polymer-PEG-targeting ligand conjugates and nanoparticles comprising a polymer-PEG targeting ligand conjugate. PEGylation enhances the circulatory half-life by reducing clearance of the nanoparticles by the reticuloendothelial (RES) system.

In some of those embodiments, the surface comprises a polymer-PEG conjugate at a concentration of about 4 mole percent to about 15 mole percent of the surface, including, but not limited to, about 4 mole percent, about 5 mole percent, about 6 mole percent, about 7 mole percent, 8 mole percent, about 9 mole percent, about 10 mole percent, about 1 1 mole percent, about 12 mole percent, about 13 mole percent, about 14 mole percent, and about 15 mole percent PEG. Higher percentage values (expressed in mole percent) of PEG have also been found to be useful. Useful mole percent values include those from about 12 mole percent to about 50 mole percent. Preferably, the values are from about 15 mole percent to about 40 mole percent. Also preferred are values 1 from about 15 mole percent to about 35 mole percent. Most preferred values are from about 20 mole percent to about 25 mole percent, for example 23 mole percent.

The polyethylene glycol moiety of the lipid-PEG conjugate can have a molecular weight ranging from about 100 to about 20,000 g/mol, including but not limited to about 100 g/mol, about 200 g/mol, about 300 g/mol, about 400 g/mol, about 500 g/mol, about 600 g/mol, about 700 g/mol, about 800 g/mol, about 900 g/mol, about 1000 g/mol, about 5000 g/mol, about 10,000 g/mol, about 15,000 g/mol, and about 20,000 g/mol. In some embodiments, the lipid-PEG conjugate comprises a PEG molecule having a molecular weight of about 2000 g/mol.

### Therapeutic uses of the conjugates of the invention

The mRNA of the invention preferably encodes a protein that treats, or contributes to the treatment, of a disease. Upon delivery of the composition of the invention to the target liver cell, the mRNA of the invention can be translated into its encoded protein. In this sense, a preferred embodiment refers to the composition of the invention for use in medicine. The above mentioned disease can be a disease affecting the liver or affecting any organ, structure of function of the body. Thus, in yet another embodiment, the invention relates to the composition of the invention for use in the treatment of a disease which requires the expression in the liver of the protein encoded by the mRNA of the invention.

Non-limiting examples of diseases to be treated include: erythropoietin deficiency, Alpha-1 antitrypsin deficiency, mucopolysaccharidosis type I (Hurler syndrome), mucopolysaccharidosis type II (Hunter syndrome), mucopolysaccharidosis type IV (Morquio syndrome A and B), mucopolysaccharidosis type VI (Maroteaux-Lamy syndrome), mucopolysaccharidosis type VII (Sly Syndrome), lysosomal acid alpha-glucosidase enzime deficiency (Pompe's disease), glucocerebrosidase deficiency(Gaucher disease type I), adenosine deaminase enzime deficiency, Glanzmann-Riniker syndrome, Urea cycle disorder Ornithine transcarbamylase, Urea cycle disorder N-acetylglutamate synthase deficiency, Urea cycle disorder carbamoyl phosphate synthetase deficiency, Urea cycle disorder argininosuccinate synthase deficiency (citrullinemia), Urea cycle disorder argininosuccinate lyase deficiency (argininosuccinate aciduria), Urea cycle disorder arginase deficiency (argininemia), Lysosomal acid lipase deficiency (Wolman disease), Ceramidase deficiency (Farber disease), alpha galactosidase A enzime deficiency (Fabry's disease), alpha-N-acetylgalactosaminidase deficiency (Shindler disease or Knazaki disease), acid maltase deficiency (Pompe disease), Hemophilia (Factor VIII, IX deficiency or other rare cases such as Factor I, II, V, VII, X, XI, XII and XIII deficiency), Cancer (mABs: from table below).

| **Antibody** | **Target** | **FDA-Approved indication** | **Mechanism of action** |
|---|---|---|---|
| Trastuzumab (Herceptin®) humanized IgG1 | HER2 (ErbB2) | HER2-positive breast cancer, as single agent or in combination with chemotherapy for (i) adjuvant or (ii) palliative treatment; HER2-possitive gastric or gastroesophageal junction carcinoma, as first-line treatment in combination with cisplatin and capecitabine/5 -FU | Inhibition of HER2 signaling; ADCC |
| Bevacizumab (Avastin®) humanized IgG1 | VEGF | For the palliative treatment of colorectal cancer, non-squamous non-small cell lung cancer, glioblastoma o renal cell carcinoma | Inhibition of VEGF signaling |
| Cetuximab (Erbitux®) chimeric human/murine IgG1 | EGFR (ErbB1) | In combination with radiation therapy for the initial treatment of locally or regionally advanced squamous cell cancer of the head and neck (SCCHN); As a single agent for SCCHN patients with whom prior platinum-based therapy has failed; Palliative treatment of pre-treated metastatic EGFR-positive colorectal cancer | Inhibition of EGFR signaling; ADCC |
| Panitumumab (Vectibix®)* human IgG2 | EGFR (ErbB1) | As a single agent for the treatment of pre-treated EGFR-expressing, metastatic colorectal carcinoma | Inhibition of EGFR signaling |
| Ipilimumab (Yerboy®) IgG1 | CTLA-4 | For the treatment of unresectable or metastatic melanoma | Inhibition of CTLA-4 signaling |
| Rituximab (Rituxan® and Mabthera® chimeric human/murine IgG1 | CD20 | For the treatment of CD20-positive B cell non-Hodgkin lymphoma (NHL) and chronic lymphocytic leukemia (CLL), and for maintenance therapy for untreated follicular CD20-positive NHL | ADCC; direct induction of apoptosis; CDC |
| Alemtuzumab (Campath®) humanized IgG1 | CD52 | As a single agent for the treatment of B cell CLL | Direct induction of apoptosis; CDC |
| Ofatumumab (Arzerra®) human IgG1 | CD20 | Treatment of patients with CLL refractory to fludarabine and alemtuzumab | ADCC; CDC |
| Gemtuzumab ozogamicin (Mylotarg®) humanized IgG4 | CD33 | For the treatment of patients with CD33-positive acute myeloid leukemia in first relapse who are 60 years of age or older and who are not considered candidates for other cytotoxic chemotherapy (withdrawn from use in June 2010) | Delivery of toxic payload, calicheamicin toxin |
| Brentuximab vedotin (Adcetris®) chimeric IgG1 | CD30 | For the treatment of relapsed o refractory Hodgkin lymphoma and systemic anaplastic lymphoma | Delivery of toxic payload, auristatin toxin |
| ⁹⁰Y-Ibritumomab Tiuxetan (Zevalin®) murine IgG1 | CD20 | Treatment of relapsed o refractory, low-grade, or follicular B cell NHL; Previously untreated follicular NHL in patients who achieve a partial or complete response to first-line chemotherapy | Delivery of the radioisotope yttrium-90 |
| ¹³¹I-Tositumomab (Bexxar®) murine IgG2 | CD20 | Treatment of patients with CD20 antigen-expressing relapsed o refractory low-grade, follicular, or transformed NHL | Delivery of the radioisotope iodine-131; ADCC; direct induction of apoptosis |

| | | | |
|---|---|---|---|
| * Not recommended in colorrectal cancer patients whose tumors express mutated KRas | | | |

Non-limiting examples of mRNAs that could be part of the composition of the invention for use in the treatment of each of said disease are indicated in Table 1.

**Table 1. List of proteins that can be encoded by the mRNA of the composition of the invention for use in the treatment of a disease. The disease/s that can be treated with each encoded protein are/is indicated on the right column of the corresponding protein.**

| **Protein to be encoded by an mRNA for use in the treatment of the disease indicated on the right column** | **Disease to be treated with the mRNA encoding the protein indicated on the left column** |
|---|---|
| erythropoietin (EPO) | Erythropoietin deficiency (EPO) |
| Alpha-1 antitrypsin (AATD) | Alpha-1 antitrypsin deficiency (AATD) |
| alpha-L-iduronidase | Mucopolysaccharidosis type I (MPS I; Hurler syndrome) |
| iduronate-2-sulfatase (I2S) | Mucopolysaccharidosis type II (MPS II; Hunter syndrome) |
| A: GALNS gene product (galactosamine-6 sulfatase), B: GLB1 gene product (beta-galactosidase) | Mucopolysaccharidosis type IV (MPS IV; Morquio syndrome A and B) |
| Arylsulfatase B (N-acetylgalactosamine-4-sulfatase, chondroitinsulfatase, chondroitinase, acetylgalactosamine 4-sulfatase, N-acetylgalactosamine 4-sulfate sulfohydrolase, EC 3.1.6.12) | Mucopolysaccharidosis type VI (MPS VI; Maroteaux-Lamy syndrome) |
| beta-glucuronidase | Mucopolysaccharidosis type VII (MPS VII; Sly Syndrome) |
| acid alpha-glucosidase | lysosomal acid alpha-glucosidase enzime deficiency (Pompe's disease) |
| glucocerebrosidase | glucocerebrosidase deficiency(Gaucher disease type I) |
| adenosine deaminase | adenosine deaminase enzime deficiency (ADA-SCID), Glanzmann-Riniker syndrome |
| Ornithine transcarbamylase (OTC) | Urea Cycle disorders: Ornithine transcarbamylase, or OTC, deficiency. |
| N-acetylglutamate synthase | Urea Cycle disorders: N-acetylglutamate synthase deficiency |
| Carbamoyl phosphate synthetase (CPS I) | Urea Cycle disorders: Carbamoyl phosphate synthetase (CPS I) deficiency: |
| Argininosuccinate synthase | Urea Cycle disorders: Argininosuccinate synthase deficiency - citrullinemia (citrullinuria) |
| Argininosuccinate lyase | Urea Cycle disorders: Argininosuccinate lyase deficiency (argininosuccinate aciduria) |
| Arginase | Urea Cycle disorders: Arginase deficiency (argininemia) |
| lipase A, lysosomal acid, cholesterol esterase | Lysosomal acid lipase deficiency (or LAL deficiency or LAL-D or Wolman disease) |
| ceramidase | Ceramidase deficiency (Farber disease) |
| alpha galactosidase A | alpha galactosidase A enzime deficiency (Fabry's disease) |
| alpha-N-acetylgalactosaminidase | alpha-NAGA (alpha-N-acetylgalactosaminidase) deficiency (Shindler disease or Knazaki disease) |
| acid alpha-glucosidase (acid maltase). | acid alpha-glucosidase (acid maltase deficiency or Pompe disease) |
| Factor VIII and Factor IX And for rare cases: Factor I, II, V, VII, X, XI, XII and XIII | Hemophilia (Factor VIII or IX deficiency) and Factor I, II, V, VII, X, XI, XII and XIII deficiency |
| mABs | Cancer treatment: Cancer (mABs: from table below). |

| **Antibody** | **Target** | **FDA-Approved indication** | **Mechanism of action** |
|---|---|---|---|
| Trastuzumab (Herceptin®) humanized IgG1 | HER2 (ErbB2) | HER2-positive breast cancer, as single agent or in combination with chemotherapy for (i) adjuvant or (ii) palliative treatment; HER2-possitive gastric or gastroesophageal junction carcinoma, as first-line treatment in combination with cisplatin and capecitabine/5- FU | Inhibition of HER2 signaling; ADCC |
| Bevacizumab (Avastin®) humanized IgG1 | VEGF | For the palliative treatment of colorectal cancer, non-squamous non-small cell lung cancer, glioblastoma o renal cell carcinoma | Inhibition of VEGF signaling |
| Cetuximab (Erbitux®) chimeric human/murine IgG1 | EGFR (ErbB1) | In combination with radiation therapy for the initial treatment of locally or regionally advanced squamous cell cancer of the head and neck (SCCHN); As a single agent for SCCHN patients with whom prior platinum-based therapy has failed; Palliative treatment of pre-treated metastatic EGFR-positive colorectal cancer | Inhibition of EGFR signaling; ADCC |
| Panitumumab (Vectibix®)* human IgG2 | EGFR (ErbB1) | As a single agent for the treatment of pre-treated EGFR-expressing, metastatic colorectal carcinoma | Inhibition of EGFR signaling |
| Ipilimumab (Yerboy®) IgG1 | CTLA-4 | For the treatment of unresectable or metastatic melanoma | Inhibition of CTLA-4 signaling |
| Rituximab (Rituxan® and | CD20 | For the treatment of CD20-positive B cell non-Hodgkin lymphoma | ADCC; direct induction of apoptosis; |
| Mabthera® chimeric human/murine IgG1 | | (NHL) and chronic lymphocytic leukemia (CLL), and for maintenance therapy for untreated follicular CD20-positive NHL | CDC |
| Alemtuzumab (Campath®) humanized IgG1 | CD52 | As a single agent for the treatment of B cell CLL | Direct induction of apoptosis; CDC |
| Ofatumumab (Arzerra®) human IgG1 | CD20 | Treatment of patients with CLL refractory to fludarabine and alemtuzumab | ADCC; CDC |
| Gemtuzumab ozogamicin (Mylotarg®) humanized IgG4 | CD33 | For the treatment of patients with CD33-positive acute myeloid leukemia in first relapse who are 60 years of age or older and who are not considered candidates for other cytotoxic chemotherapy (withdrawn from use in June 2010) | Delivery of toxic payload, calicheamicin toxin |
| Brentuximab vedotin (Adcetris®) chimeric IgG1 | CD30 | For the treatment of relapsed o refractory Hodgkin lymphoma and systemic anaplastic lymphoma | Delivery of toxic payload, auristatin toxin |
| ⁹⁰Y-Ibritumomab Tiuxetan (Zevalin®) murine IgG1 | CD20 | Treatment of relapsed o refractory, low-grade, or follicular B cell NHL; Previously untreated follicular NHL in patients who achieve a partial or complete response to first-line chemotherapy | Delivery of the radioisotope yttrium-90 |
| ¹³¹I-Tositumomab (Bexxar®) murine IgG2 | CD20 | Treatment of patients with CD20 antigen-expressing relapsed o refractory low-grade, follicular, or transformed NHL | Delivery of the radioisotope iodine-131; ADCC; direct induction of apoptosis |

| | | | |
|---|---|---|---|
| * Not recommended in colorrectal cancer patients whose tumors express mutated KRas | | | |

The mRNA of the composition of the invention can encode proteins required for the proper function of the liver of the subject to be treated, or proteins required for the proper function of any other organ or function of its body, independently on whether the protein is synthesized in a healthy subject in the liver or in any other organ. Said protein may reach the target part of the body where it elicits its therapeutic function systemically.

The method of administration of the composition of the invention will depend on factors such as the duration of the therapy and whether the composition will be administered for preventing or treating purposes. Thus, the composition of the invention can be administered chronically, sub-chronically or acutely.

The term "chronically", as used herein, refers to a method of administration wherein the composition is administered continuously to the patient for extended periods of time in order to maintain the therapeutic effect during this period. Chronic administration forms include the daily administration of one or multiple doses of the composition, twice daily, three times daily or more frequently. Chronic administration includes also repeated administrations during multiple weeks. Rest periods can be used as part of chronic dispensation. Alternatively, chronic administration can involve the administration as a bolus or by continuous transfusion or subcutaneously which can be performed daily, every two days, every 3 to 15 days, every 10 days or more. Typically, chronic administration is continued for at least one week, at least 7 weeks, at least 8 weeks, at least 9 weeks, at least 10 weeks, at least 11 weeks, at least 12 weeks, at least four months, at least 5 months, at least 6 months, at least 9 months, at least one year, at least two years or more.

The composition may be administered by any suitable administration route, such as, but not limited to, parenteral, oral, topical, nasal or rectal route. In a particular embodiment, the composition is administered intrahepatically, intraportally, intraperitoneally, intravenously, subcutaneously, intradermically or intramuscularly. In a preferred embodiment, the composition of the invention is administered intrahepatically or intraportally.

The translation of the mRNA of the complex of the invention in the liver target cell after administration of the composition of the invention can be determined by any suitable technique known by the skilled person, for example by microscopy or immunohistochemistry.

The following examples and drawings are provided by way of illustration, and are not intended to be limiting of the present invention.

### EXAMPLES

### eGFP mRNA-15U-GalNAc complex formation

Enhanced Green Fluorescent Protein (eGFP) (996 bp) with 5-methylcytidine and pseudouridine modifications (CleanCap™EGFP mRNA (5moU); ref: L-7201; TriLink Biotechnologies) and the oligonucleotide poliU-GalNAc (Figure 1) were mixed in equimolar concentration 1:10 or 1:20 in duplex buffer (100 mM potassium acetate, 30 mM HEPES, pH 7.5, IDT Integrated DNA Technologies), heated at 94°C for 2 min and gradually cooled down. Solution was prepared and used the same day in RNase free conditions. Final dilution for administration was prepared in PBS buffer.

### Cas9 mRNA-15U-GalNAc complex formation

Cas9 mRNA (4,5 kb) including N and C terminal nuclear localization signal with 5-methylcytidine and pseudouridine modifications. CleanCap™ CRISPR Associated Protein 9 mRNA (U-modified); ref: L7206; TriLink Biotechnologies) and the oligonucleotide poliU-GalNAc (Figure 1) were mixed in equimolar concentration 1:10 or 1:20 in duplex buffer (100 mM potassium acetate, 30 mM HEPES, pH 7.5, IDT Integrated DNA Technologies), heated at 94°C for 2 min and gradually cooled down. Solution was prepared and used the same day in RNase free conditions. Final dilution for administration was prepared in PBS buffer.

### Animal administration

Mice C57BL/6 were administered in the tail vain with PBS or e-GFP-mRNA, eGFP-mRNA-15U-GalNAc complex or Cas9 mRNA-15U-GalNAc at a concentration of 100 ng in single dose and sacrificed at 8 and 72 hrs. Animals were anesthetized with ketamine/medetomidine cardiac perfused with cold PBS. The liver was extracted, the lobes separated and the caudate lobe was selected for further analysis.

### mRNA detection

Total RNA was extracted from the caudate lobe of the liver with Norgen Total-RNA purification kit (Norgen) in a RNase free environment using a TissueLyser II (Qiagen) for mechanical disruption of the tissue. Genomic DNA was eliminated by digestion with RNase free DNase set (Qiagen) and final RNA was eluted form the column with elution buffer and centrifugation (2min x 200g + 2 min x 14000g). Final quantification and quality assessment was evaluated by spectrophotometry (Nanodrop) by analysis of the ratios of absorbance at 260/280 and 269/230 nm.

For eGFP mRNA evaluation we performed RT-PCR (reverse transcription-polymerase chain reaction) with primers designed to amplify a fragment of 250 pb. RT was performed with SuperScript VILO cDNA synthesis kit (Fisher) with random primers using 200 µg of starting RNA and incubated in a C1000 ThermoCycler (BioRad) with 4 µl of Superscript retrotransciptase. Once cDNA was obtained it was stored at -20°C. cDNA samples were amplified to detect eGFP-mRNA with Q5 DNA polymerase (NEB) at a concentration of 2U/µl and 34 cycles of annealing with the following primers: forward primer: ACGTAAACGGCCACAAGTTC, reverse primer: CTTGTACAGCTCGTCCATGC. The PCR reaction was carried out in a C1000 ThermoCycler (BioRad). 10 µl of the final reaction were visualized by agarose gel electrophoresis (Figure 2).

### Protein detection

Protein detection was analyzed by confocal microscopy of caudate lobe of treated animals. Liver samples were cryosected at 7 µm and stained with DAPI (4,6-Diamidine-2-phenylindole dihydrochloride, Sigma) at a concentration of 300 nM in PBS buffer for 5 min for visualization of the cellular nucleus. eGFP was detected by direct visualization using confocal microscopy (Leica confocal microscope SP5) with laser excitation at 488 nm and Cas9 was detected using the antibody:Anti-CRISPR-Cas9 antibody [7A9-3A3] (ab191468) (1:500).

### Liver-specific expression of eGFP

As shown in Figures 3 and 4, eGFP expression can be detected in liver cells of mice that were administered with the eGFP-mRNA complexed to a 15-mer polyU-GalNAc.

### Liver-specific expression of Cas9

As shown in figure 5, Cas9 expression can be detected in liver cells of mice that have been administered with Cas9-mRNA complexed to a 15-mer polyU-GalNAc.

### SEQUENCE LISTING

### NLS:

3- PKKKRKV
4- KRPAATKKAGQAKKKK
5- PAAKRVKLD
6- RQRRNELKRSP
7- NQSSNFGPMKGGNFGGRSSGPYGGGGQYFAKPRNQGGY
8- RMRIXFKNKGKDTAELRRRRVEVSVELRKAKKDEQILKRRNV
9- VSRKRPRP
10- PPKKARED
11- PQPKKKPL
12- SALIKKKKKMAP
13- DRLRR
14- PKQKKRK
15- RKLKKKIKKL
16- REKKKFLKRR
17- KRKGDEVDGVDEVAKKKSKK
18- RKCLQAGMNLEARKTKK
19- MAPKKKRKVGIHRGVP
20- PKKKRKVEDPKKKRKVD

### CPPS

### Table 1 CPPs derived from heparan binding proteins

21- RKKRRRESRKKRRRES
22- GRPRESGKKRKRKRLKP
23- GKRKKKGKLGKKRDP
24- GKRKKKGKLGKKRPRSR
25- RKKRRRESRRARRSPRHL
26- SRRARRSPRESGKKRKRKR
27- VKRGLKLRHVRPRVTRMDV
28- SRRARRSPRHLGSG
29- LRRERQSRLRRERQSR
30- GAYDLRRRERQSRLRRRERQSR
CPPs derived from RNA binding proteins
31- RKKRRQRRR
32- RRRRNRTRRNRRRVR
33- TROARRNRRRRWREROR
34- TRRQRTRRARRNR
35- KMTRAQRRAAARRNRWTAR
36- NAKTRRHERRRKLAIER
37- MDAQTRRRERRAEKQAQWKAAN
38- TAKTRYKARRAELIAERR
39- TRRNKRNRIQEQLNRK
CPPs derived from DNA binding proteins
40- PRRRRSSSRPVRRRRRPRVSRRRRRRGGRRRR
41- RIKAERKRMRNRIAASKSRKRKLERIAR
42- KRRIRRERNKMAAAKSRNRRRELTDT
43- KRARNTEAARRSRARKLQRMKQ

### CPPs derived from heparan-, RNA- and DNA binding proteins

44- RQIKIWFQNRRMKWKK
45- RVIRVWFQNKRCKDKK
46- 
SKRTRQTYTRYQTLELEKEFHFNRYITRRRRIDIANALSLSERQIKIWFQN
RRMKSKKDR
47- SQIKIWFQNKRAKIKK
48- RQVTIWFQNRRVKEKK
49- KQINNWFINQRKRHWK
50- RHIKIWFQNRRMKWKK

### Table 2. CPPs derived from signal peptides

Amphipathic (I), signal peptide + NLS
51- MGLGLHLLVLAAALQGAKKKRKV
52- MVKSKIGSWILVLFVAMWSDVGLCKKRPKP
53- MANLGYWLLALFVTMWTDVGLCKKRPKP
Hydrophobic, signal peptide alone
54- AAVLLPVLLAAP

### Table 3. CPPs derived from antimicrobial peptides

55- RRIRPRPPRLPRPRPRPLPFPRPG
56- VDKGSYLPRPTPPRPIYNRN
57- KCFQWQRNMRKVRGPPVSCIKR
58- TRSSRAGLQWPVGRVHRLLRK
59- GIGAVLKVLTTGLPALISWIKRKRQQ
60- GIGKWLHSAKKFGKAFVGEIMNS
61- LLGDFFRKSKEKIGKEFKRIVQRIKDFLRNLVPRTE
62- RGGRLSYSRRRFSTSTGR
63- YKQCHKKGGKKGSG
64- ALWKTLLKKVLKAPKKKRKV
65- HARIKPTFRRLKWKYKGKFW

### Table 4. CPPs derived from viral proteins.

66- TKRRITPKDVIDVRSVTTEINT
67- RQGAARVTSWLGRQLRIAGKRLEGRSK
68- NAATATRGRSAASRPTQRPRAPARSASRPRRPVQ
69- RHSRIGIIQQRRTRNG
70- KLIKGRTPIKFGKADCDRPPKHSQNGMGK
71- PLSSIFSRIGDP
72- DPKGDPKGVTVTVTVTVTGKGDPKPD

### Table 5. CPPs derived from various natural proteins

73- RRIPNRRPRR
74- RLRWR
75- MVRRFLVTLRIRRACGPPRVRV
76- MVTVLFRRLRIRRACGPPRVRV
77- LLIILRRRIRKQAHAHSK
78- LSTAADMOGVVTDGMASG
79- LSTAADMQGVVTDGMASGLDKDYLKPDD
80- KFHTFPQTAIGVGAP
81- VPTLK
82- PMLKE
83- VPALR
84- VSALK
85- IPALK
86- PFVYLI
87- PIEVCMYREP

### Table 6. Designed CPPs and CPPs derived from peptide libraries.

88- R8
89- R9
90- R10
91- R12
92- KETWWETWWTEWSQPKKRKV
93- GLAFLGFLGAAGSTMGAWSOPKKKRKV
94- GWTLNSAGYLLGKINLKALAALAKKIL
95- AGYLLGHINLHHLAHLAibHHIL
96- KLALKALKALKAALKLA
97- RRWWRRWRR
98- GLWRALWRLLRSLWRLLWRA
99- LIRLWSHLIHIWFQNRRLKWKKK
100- WEAALAEALAEALAEHLAEALAEALEALAA
101- WEAKLAKALAKALAKHLAKALAKALKACEA
102- LKTLTETLKELTKTLTEL
103- QLALQLALQALQAALQLA
104- (PPR)3
105- (PPR)4
106- (PPR)5
107- (PPR) 6
108- (PRR)3
109- (PRR)4
110- (PRR)5
111- (PRR) 6
112- GPSQPTYPGDDAPVRDLIRFYRDLQRYLNVVTRHRY
113- GPSQPTYPGDDAPVRDLIRFYRDLRRYLNVVTRHRY
114- GPSQPTYPGDDAPVRDLRRFYRDLRRYLNVVTRHRY
115- G(PLXX)nPL, where PL is a proline based mimic of leucine, and X is a proline-based mimic of lysine or arginine
116- VRLPPPVRLPPPVRLPPP
117- VELPPPVELPPPVELPPP
118- FKIYDKKVRTRVVKH
119- RASKRDGSWVKKLHRILE
120- KGTYKKKLMRIPLKGT
121- LYKKGPAKKGRPPLRGWFH
122- HSPIIPLGTRFVCHGVT
123- YTAIAWVKAFIRKLRK
124- IAWVKAFIRKLRKGPLG
125- RLSGMNEVLSFRWL
126- SDLWEMMMVSLACQY
127- VTWTPQAWFQWV
128- GSPWGLQHHPPRT
129- GPFHFYQFLFPPV
130- TSPLNIHNGQKL
131- CAYHRLRRC
132- RCGRASRCRVRWMRRRRI
133- PYSRPHVQLWYPNRESCRSLIRSLGP
134- PLILLRLLRGQF
135- PLIYLRLLRGQF
136- KLWMRWYSPTTRRYG

### Fusogenic peptides

137- influenza HA-2 peptide, GLFGAIAGFIENGWEGMIDGWYG
138- melittin, GIGAVLKVLTTGLPALISWIKRKRQQ
139- tat (48-60), GRKKRRQRRRPPQ
140- penetratin, RQIKIWFQNRRMKWKK
141- transportan, GWTLNSAGYLLGKINLKALAALAKKIL
142- GALA peptide, WEAALAEALAEALAEHLAEALAEALEALAA
143- KALA peptide, WEAKLAKALAKALAKHLAKALAKALKACEA
144- JST-1, GLFEALLELLESLWELLLEA
145- ppTGl, GLFKALLKLLKSLWKLLLKA
146- ppTG20, GLFRALLRLLRSLWRLLLRA
147- CHK6HC
148- HSWYG
149- GLFHAIAHFIHGGWHGLIHGWYG
150- ppTG21: GLFHALLHLLHSLWHLLLHA

### Nuclear localization sequence

151- NLS of the SV 40 virus large T-antigen PKKKRKV
152- nucleoplasmin bipartite NLS KRPAATKKAGQAKKKK
153- c-myc NLS PAAKRVKLD
154- c-myc NLS RQRRNELKRSP
155- hRNPA1 M9 NLS
NQSSNFGPMKGGNFGGRSSGPYGGGGQYFAKPRNQGGY
156- IBB domain from importinalpha
RMRIXFKNKGKDTAELRRRRVEVSVELRKAKKDEQILKRRNV (wherein X is any amino acid)
157- Sequence from the myoma T protein VSRKRPRP
158- Sequence from the myoma T protein PPKKARED
159- Sequence from human p53 PQPKKKPL
160- Sequence of mouse c-abl IV SALIKKKKKMAP
161- Sequence of the influenza virus NS1 DRLRR
162- Sequence of the influenza virus PKQKKRK
163- Sequence of the Hepatitis virus delta antigen RKLKKKIKKL
164- Sequence of the mouse Mx1 protein REKKKFLKRR
165- Sequence of the human poly(ADP-ribose) polymerase
KRKGDEVDGVDEVAKKKSKK
166- Sequence of the steroid hormone receptors (human) glucocorticoid RKCLQAGMNLEARKTKK
167- MAPKKKRKVGIHRGVP
168- PKKKRKVEDPKKKRKVD

## Claims

1. A complex for selective delivery of an mRNA to a liver cell comprising:
a) an mRNA and
b) one or more conjugates having the general formula:
N-Tx
wherein N is a nucleic acid, the sequence of which is at least in part substantially complementary to a region of the mRNA, T is a liver targeting moiety comprising at least one selectivity agent S which binds specifically to one or more proteins on the surface of a liver cell and x is an integer which is equal to or higher than 1, wherein a complex is formed between the mRNA and the nucleic acid N by base pairing between the region within the mRNA which is complementary to the nucleic acid and the nucleic acid forming part of the conjugate.

2. The complex according to claim 1 comprising a plurality of conjugates having the general formula N-Tx wherein the nucleic acids within each conjugate bind to different regions within the mRNA.

3. The complex according to any of claims 1 or 2, wherein the complex formed between the nucleic acid and the region within the mRNA complementary to the nucleic acid comprises interstrand covalent bonds between reactive groups of at least one nucleotide from the nucleic acid N and at least one nucleotide of the mRNA.

4. The complex according to claim 3 wherein the covalent bonds are formed by psolaren cross-link.

5. The complex according to claim 4 wherein the psolaren moiety is covalently linked to the 5' and/or the 3' end of the nucleic acid of the complex and wherein the psolaren cross-link reaction covalently links the psolaren moiety and a pyrimidine nucleotide of the mRNA of the complex.

6. The complex according to any of claims 1 to 5 wherein within each of the conjugates, the targeting moiety or moieties are connected to the nucleic acid via a terminal nucleotide position and/or via an internal nucleotide position.

7. The complex according to claims 1 to 6 wherein the nucleic acid N which forms part of at least one of the conjugates comprises a polyuridine or poly-thymidine region and wherein a complex is formed by base pairing between said polyuridine or poly-thymidine and the polyA+ tail within the mRNA.

8. The complex according to claim 7 wherein the complex comprises a first conjugate, wherein the nucleic acid within said first conjugate comprises a polyuridine or poly-thymidine region and at least a second conjugate wherein the nucleic acid has a sequence which is substantially complementary to a region of the mRNA which is not the polyA+ region wherein a complex is formed by base pairing between the polyuridine or poly-thymidine within the nucleic acid of the first conjugate and the polyA+ tail within the mRNA and by base pairing between the nucleic acid of the second conjugate and the region which is not the polyA+ tail within the mRNA.

9. The complex according to any of claims 1 to 8 wherein the nucleic acid within one or more of the conjugates comprises at least 15 nucleotides.

10. The complex according to any of claim 1 to 7 wherein the mRNA contains:
a) an Anti-reverse cap analog (ARCA),
b) One or more nucleosides selected from the group consisting of 2'-O-methyl nucleoside, 2'-fluoronucleoside, pseudouridine, N-methyl pseudouridine, 2-thiouridine, 5-methylcytidine, 6-methyl adenosine and inosine, or
c) a combination of a) and b)

11. The complex according to any of claims 1 to 8 wherein the mRNA encodes a protein which is naturally expressed in a liver cell.

12. The complex according to any of claims 1 to 11 wherein each targeting moiety comprises n selectivity agents S1 to Sn, wherein the selectivity agents S1 to Sn are the same or different.

13. The complex according to any of claims 1 to 10 wherein n is equal to or higher than 2 and wherein the selectivity agents S1 to Sn are connected to the nucleic acid via a single branched spacer molecule containing the selectivity agents S1 to Sn connected to different branches of the spacer molecule.

14. The complex according to any of claims 1 to 13, wherein at least one of the selectivity agents S₁ to Sn is a ligand of the asialoglycoprotein receptor.

15. The complex according to claim 14 wherein the ligand of the asialoglycoprotein receptor comprises a GalNAc or galactose moiety.

16. The complex according to claim 15 wherein the ligand of the asialoglycoprotein receptor is a triantennary GalNAc moiety.

17. The complex of any of claims 1 to 16 further comprising a detectable moiety attached to the nucleic acid which forms part of the conjugate.

18. The complex according to any one of the preceding claims for use in medicine.

19. The complex according to any of claims 1 to 17 for use in the treatment of a disease which requires the expression in the liver of the protein encoded by the mRNA forming part of the conjugates.

20. The complex for use according to claim 19 wherein the mRNA is selected from the group shown in the first column in Table 1 and the disease to be treated is selected from the group shown in the second column in Table 1.

21. The complex for use according to any of claims 18 to 21, wherein the composition is administered to a human subject and the dose of each administration of the composition comprises 4 µg of the mRNA forming part of the complex per Kg of the human subject.

22. A method for preparing a complex according to any of claims 1 to 17 comprising contacting the mRNA and the conjugate or conjugates under conditions adequate for hybridization between the mRNA and the nucleic acid or nucleic acids within the conjugate or conjugates.
